# EUROPÄISCHE PATENTSCHRIFT

(11) **EP 1 213 013 B1**
(45) Veröffentlichungstag und Bekanntmachung des Hinweises auf die Patenterteilung: **04.04.2007**
(21) Anmeldenummer: 01126756.4
(22) Anmeldetag: 09.11.2001
(51) Int. Cl.: A61K 9/18, A61K 9/10, A61K 47/42, A23L 1/305, A23K 1/00, A23K 1/16, A61K 8/00

(54) **Verfahren zur Herstellung fester Zubereitungen wasserlöslicher, schwer wasserlöslicher oder wasserunlöslicher Wirkstoffe**
Process for preparing solid compositions of water-soluble, hardly water-soluble or water-insoluble active agents
Procédé de préparation de compositions solides d' agents actifs solubles, difficilement solubles ou insolubles dans l'eau

(30) Priorität: 29.11.2000 DE 10059231; 22.06.2001 DE 10129713
(43) Veröffentlichungstag der Anmeldung: 12.06.2002
(73) Patentinhaber: BASF Aktiengesellschaft, 67056 Ludwigshafen (DE)
(72) Erfinder: Auweter, Helmut, Dr., 67117 Limburgerhof (DE); Bohn, Heribert, 67319 Wattenheim (DE); Lüddecke, Erik, Dr., 67112 Mutterstadt (DE); Hinz, Willy, Dr., 68199 Mannheim (DE); Runge, Frank, Dr., 67159 Friedelsheim (DE); Pfeiffer, Angelika-Maria, Dr., 67134 Birkenheide (DE)

(56) Entgegenhaltungen:
- EP-A- 0 065 193
- EP-A- 0 278 284
- EP-A- 0 845 503
- EP-A- 0 937 412
- WO-A-93/10768
- WO-A-94/19411
- DE-A- 19 919 751
- GB-A- 393 319

## Beschreibung

Die Erfindung betrifft ein Verfahren zur Herstellung fester Zubereitungen mindestens eines, für den Lebensmittel- und Tierfuttermittelbereich oder für pharmazeutische und kosmetische Anwendungen geeigneten, wasserlöslichen, schwer wasserlöslichen oder wasserunlöslichen Wirkstoffs. Ferner betrifft die Erfindung ölige Suspensionen, in denen diese Zubereitungen enthalten sind und deren Verwendung als Zusatz zu Tierfuttermitteln, Lebensmitteln, Pharmazeutika und kosmetischen Präparaten.

Zahlreiche für den Lebensmittel- und Tierfuttermittelbereich oder für pharmazeutische und kosmetische Anwendungen geeignete Wirkstoffe, beispielsweise fettlösliche Vitamine, Carotinoide aber auch die natürlichen Farbstoffe Curcumin oder Carmin sind aufgrund ihrer Wasserunlöslichkeit sowie ihrer Oxidationsempfindlichkeit nur in Form speziell stabilisierter Zubereitungen einsetzbar. Eine direkte Verwendung der kristallinen Materialien u.a. zum Färben von wässrigen Lebensmitteln, als Futterzusätze oder als Wirkstoffe in kosmetischen Zubereitungen ist in der Regel nicht möglich. Die hohen Anforderungen hinsichtlich Bioverfügbarkeit, Färbungseigenschaften sowie Dispergierbarkeit insbesondere in wässrigen aber auch in lipophilen Medien sind nur mittels spezieller Formulierungen zu erfüllen.

Nur durch Zubereitungen, in denen die Wirkstoffe, beispielsweise Carotinoide in fein verteilter Form und gegebenenfalls durch Schutzkolloide oxidationsgeschützt vorliegen, lassen sich bei der direkten Einfärbung von Lebensmitteln zufriedenstellende Farbausbeuten erzielen. Diese in Tierfuttermitteln verwendeten Formulierungen führen zu einer höheren Bioverfügbarkeit der Wirkstoffe und damit indirekt zu besseren Färbungseffekten z.B. bei der Eidotter- oder Fischpigmentierung.

Aus der Literatur sind bereits eine Reihe verschiedenster Formulierverfahren bekannt, die alle das Ziel haben, die Kristallitgröße der Wirkstoffe zu verkleinern und auf einen Teilchengrößenbereich von kleiner 10 µm zu bringen.

Zahlreiche Methoden, u.a. beschrieben in Chimia 21, 329 (1967), WO 91/06292 sowie in WO 94/19411, bedienen sich dabei der Vermahlung von Carotinoiden mittels einer Kolloidmühle und erzielen damit Partikelgrößen von 2 bis 10 µm.

Daneben existieren kombinierte Emulgier-/Sprühtrocknungsverfahren, wie sie z.B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen Carotinoidpräparaten dadurch, dass man beispielsweise β-Carotin in einem flüchtigen, mit Wasser mischbaren organischen Lösungsmittel bei Temperaturen zwischen 50°C und 200°C, gegebenenfalls unter erhöhtem Druck, innerhalb einer Zeit von weniger als 10 Sekunden löst. Aus der erhaltenen molekulardispersen Lösung wird das β-Carotin durch sofortiges schnelles Mischen mit einer wässrigen Lösung eines Schutzkolloids bei Temperaturen zwischen 0°C und 50°C ausgefällt. Man erhält so ein kolloid-disperses β-Carotin-Hydrosol mit orange-gelber Farbnuance. Anschließende Sprühtrocknung der Dispersion liefert ein freifließendes Trockenpulver, das sich in Wasser unter Bildung einer klaren, gelborange gefärbten Dispersion löst.

Ein analoges Verfahren zur Herstellung von feinverteilten, pulverförmigen Carotinoidpräparaten wird in EP-A-0 937 412 unter Verwendung von mit Wasser nicht mischbaren Lösungsmitteln beschrieben.

WO 98/26008 betrifft die Verwendung eines Gemisches aus niedermolekularen und hochmolekularen Schutzkolloiden zur Herstellung redispergierbarer Xanthophyll-haltige Trockenpulver.

Die Verwendung von Carotinoiden als Futtermittelzusatzstoffe in der Tierernährung erfolgt mehr und mehr in Form flüssiger Zubereitungen. Dies hat u.a. den Vorteil, dass die Dosierung einfacher und genauer erfolgen kann. Ferner ist es möglich, bei der sogenannten "post-pelleting-application" beispielsweise Futtermittelpellets erst nach deren Herstellung mit einer flüssigen Zubereitung von Futtermittelzusatzstoffen zu beladen. Dies hat zur Folge, dass selbst oxidations- und temperaturempfindliche Zusatzstoffe wie Carotinoide ohne größere Verluste eingesetzt werden können.

Beispiele für "post-pelleting-application" (PPA) finden sich u.a. in GB-A-2 232 573 sowie in EP-A-0 556 883 und der darin zitierten Literatur.

WO 96/23420 beschreibt ölige Suspensionen von kristallinem Astaxanthin mit einer Partikelgröße von kleiner 2 µm. Derartige Suspensionen sind u.a. durch Mahlung der Astaxanthin-Kristalle in Öl hergestellt worden. Ferner beinhaltet die Schrift die Verwendung der Suspensionen zum Beladen von extrudiertem Futtermittel nach der Extrusion. Die Stabilität derartiger Suspensionen sowie die Bioverfügbarkeit des darin enthaltenen Astaxanthins sind jedoch für viele Anwendungen nicht immer ausreichend.

Carotinoid-Emulsionen - als spezielle Form einer flüssigen Formulierung - haben häufig den Nachteil, dass sie physikalisch (Auftreten von Phasentrennung) und chemisch (Auftreten von unerwünschten Hydrolyse- und/oder Redox-Reaktionen, chemische Inkompatibilität einzelner gelöster Komponenten) instabil sind und zusätzlich häufig die Gefahr einer mikrobiologischen Kontamination auftreten kann.

In anderen Verfahren, beschrieben in WO 94/19411, wird z.B. kristallines β-Carotin in Gegenwart einer wässrigen Schutzkolloid-Lösung gemahlen und anschließend durch kurzzeitiges Erhitzen bis zum Schmelzpunkt in eine amorphe Modifikation überführt.

Auch diese Formulierung, sowie die zahlreich beschriebenen, wässrigen Carotinoid-Dispersionen und O/W-Emulsionen, in denen der Wirkstoff in Gegenwart stabilisierender Schutzkolloide vorliegt, sind ebenfalls ungeeignet, da sie mit Ölen nicht mischbar sind.

EP-A-0 845 503 beschreibt flüssige, mit Öl mischbare Carotinoid-Zubereitungen, dadurch gekennzeichnet, dass sie als doppelte Dispersionssysteme eine wässrig disperse Phase mit einem Partikeldurchmesser kleiner 100 µm, in der schutzkolloid-stabilisierte Teilchen eines oder mehrerer Carotinoide dispergiert vorliegen, in einem Öl als Dispersionsmittel enthalten.

Es war die Aufgabe der vorliegenden Erfindung, ein Verfahren zur Herstellung fester Zubereitungen von wasserlöslichen, schwer wasserlöslichen oder wasserunlöslichen Wirkstoffen vorzuschlagen. Ferner sollten Zubereitungen mit hoher Wirkstoffkonzentration zur Verfügung gestellt werden.

Es war außerdem Aufgabe der vorliegenden Erfindung, flüssige, mit Öl mischbare Carotinoid-Formulierungen bereitzustellen, die u.a. im Tierernährungsbereich zum Auftragen auf Futtermittelpellets geeignet sind.

Diese Aufgabe wurde erfindungsgemäß gelöst mit einem Verfahren zur Herstellung fester Zubereitungen mindestens eines, für den Lebensmittel- und Tierfuttermittelbereich oder für pharmazeutische und kosmetische Anwendungen geeigneten, wasserlöslichen, schwer wasserlöslichen oder wasserunlöslichen Wirkstoffs, ausgewählt aus der Gruppe, bestehend aus fettlöslichen Vitaminen, wasserlöslichen Vitaminen, mehrfach ungesättigten Fettsäuren, Carotinoiden und organischen UV-Filtersubstanzen durch
a) Lösen oder Dispergieren mindestens eines der oben genannten Wirkstoffe in einer wässrigen molekulardispersen oder kolloiddispersen Lösung eines proteinhaltigen Schutzkolloids,
b) Ausflockung des proteinhaltigen Schutzkolloids zusammen mit dem Wirkstoff aus der Dispersion
   und
c) Abtrennung des ausgeflockten Feststoffs vom Wasser und von gegebenenfalls zusätzlich verwendeten Lösungsmitteln und anschließendes Überführen in ein Trockenpulver
dadurch gekennzeichnet, dass man im Verfahrensschritt b) die Flockung durch Einstellung des pH-Wertes der Dispersion auf einen wert, der im Bereich von einer pH-Wert Einheit oberhalb und unterhalb des isoelektrischen Punktes des als Schutzkolloid verwendeten Proteins liegt auslöst.

Als Wirkstoffe, die im Rahmen der vorliegenden Erfindung für den Lebensmittel- und Tierernährungsbereich oder für pharmazeutische und kosmetische Anwendungen geeignet sind, seien beispielhaft die folgenden Verbindungen genannt:
Fettlösliche Vitamine, wie z.B. die K-Vitamine, Vitamin A und Derivate wie Vitamin A-Acetat, Vitamin A-Propionat oder Vitamin A-Palmitat, Vitamin D₂ und Vitamin D₃ sowie Vitamin E und Derivate. Vitamin E steht in diesem Zusammenhang für natürliches oder synthetisches α-, β-, γ- oder δ-Tocopherol, bevorzugt für natürliches oder synthetisches α-Tocopherol sowie für Tocotrienol. Vitamin E-Derivate sind z.B. Tocopheryl-C₁-C₂₀-Carbonsäureester wie Tocopherylacetat oder Tocopherylpalmitat.

Wasserlösliche Vitamine, wie z.B. Ascorbinsäure und deren Salze wie Natriumascorbat sowie Vitamin C-Derivate wie Natrium-, Calcium- oder Magnesium-ascorbyl-2-monophosphat oder Calcium-ascorbyl-2-polyphosphat, Calcium-pantothenat, Panthenol, Vitamin B₁ (Thiamin) - als Hydrochlorid, Nitrat oder Pyrophosphat, Vitamin B₂ (Riboflavin) und deren Phosphate, Vitamin B₆ und Salze, Vitamin B₁₂, Biotin, Folsäure und Folsäurederivate wie Tetrahydrofolsäure, 5-Methyltetrahydrofolsäure, 5-Formyltetrahydrofolsäure, Nicotinsäure und Nicotinsäureamid.

Mehrfach ungesättigte Fettsäuren, wie z.B. Linolsäure, Linolensäure, Arachidonsäure, Eicosapentaensäure, Docosahexaensäure.

Carotinoide als natürliche Lebensmittelfarbstoffe. Wasserunlösliche oder schwer wasserlösliche organische UV-Filtersubstanzen, wie z.B. Verbindungen aus der Gruppe der Triazine, Anilide, Benzophenone, Triazole, Zimtsäureamide sowie der sulfonierten Benzimidazole.

Für die kosmetischen Anwendungen als Lichtschutzmittel sind als Wirkstoffe besonders 1,3,5-Triazinderivate der Formel I zu nennen. 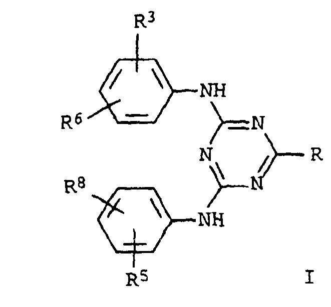 in der die Substituenten unabhängig voneinander folgende Bedeutung haben:
- R: Wasserstoff, Halogen, OH, C₁-C₂₀-Alkyl, C₁-C₂₀-Alkoxy, C₁-C₂₀-Alkoxyalkyl, C₁-C₂₀-Hydroxyalkoxy, NR¹R²,
oder einen Rest der Formel Ia 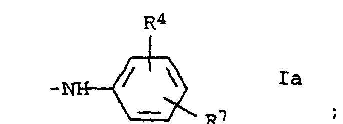
- R¹ und R²: Wasserstoff, C₁-C₂₀-Alkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- R³ bis R⁵: Wasserstoff, OH, NR⁹R¹⁰, C₁-C₂₀-Alkoxy, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- R⁶ bis R⁸: Wasserstoff, C₁-C₂₀-Alkoxy, -C(=O)-R¹¹,-C(=O)-X-R¹², SO₂R¹³, CN;
- R⁹ bis R¹¹: Wasserstoff, C₁-C₂₀-Alkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- R¹²: Wasserstoff, C₁-C₂₀-Alkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl oder einen Rest der Formel Sp-Sil;
- R¹³: C₁-C₂₀-Alkyl, C₅-C₈-Cycloalkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl;
- X: O, NR¹⁴;
- R¹⁴: Wasserstoff, C₁-C₂₀-Alkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalky;
- Sp: Spacer;
- Sil: Rest aus der Gruppe, bestehend aus Silane, Oligosiloxane und Polysiloxane.

Als Alkylreste für R, R¹ und R² sowie R⁹ bis R¹⁴ seien verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, 1,1,3-Trimethylhexyl, 1,1,3,3-Tetramethylpentyl, n-Nonyl, n-Decyl, n-Undecyl, 1-Methylundecyl, n-Dodecyl, 1,1,3,3,5,5-Hexamethylhexyl, n-Tridecyl, n-Tetradecyl, n-Pentadecyl, n-Hexadecyl, n-Heptadecyl, n-Octadecyl, n-Nonadecyl oder n-Eicosyl genannt.

Halogen für R bedeutet Fluor, Brom, Iod oder vorzugsweise Chlor.

Als Alkoxyreste für R sowie R³ bis R⁸ kommen geradkettige und verzweigte Reste mit 1 bis 20 C-Atomen, vorzugsweise mit 1 bis 12 C-Atomen, besonders bevorzugt mit 1 bis 8 C-Atomen in Betracht.

Beispielsweise sind zu nennen:

| | |
|---|---|
| Methoxy- | Ethoxy- |
| Isopropoxy- | n-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Als Hydroxyalkoxyreste für R kommen die o.g. Alkoxyreste mit zusätzlicher endständiger Hydroxylfunktion in Betracht.

Als Cycloalkylreste seien für R¹ bis R⁵ sowie R⁹ bis R¹⁴ bevorzugt verzweigte oder unverzweigte C₃-C₁₀-Cycloalkylreste wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt. Bevorzugt sind C₅-C₈-Cycloalkyl wie Cyclopentyl, Cycloheptyl, Cyclooctyl und insbesondere Cyclohexyl.

Die Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Beispiele für C₆-C₁₂-Aryl sind insbesondere Phenyl, Naphthyl und Biphenyl zu nennen.

Beispiele für C₇-C₁₀-Aralkyl sind Benzyl, Phenylethyl, α-Methylphenylethyl oder α,α-Dimethylbenzyl.

Heteroaryl-Reste sind vorteilhafterweise einfache oder kondensierte aromatische Ringsysteme mit einem oder mehreren heteroaromatischen 3- bis 7-gliedrigen Ringen. Als Heteroatome können ein oder mehrere Stickstoff-, Schwefel- und/oder Sauerstoffatome im Ring oder Ringsystem enthalten sein.

Als Substituenten der o.g. Aryl-, Aralkyl- und Heteroarylreste kommen C₁-C₄-Alkylgruppen beispielsweise Methyl, Ethyl, n-Propyl, 1-Methylethyl, n-Butyl, 1-Methylpropyl-, 2-Methylpropyl, 1,1-Dimethylethyl in Frage.

Der Begriff Spacer für Sp bedeutet in diesem Zusammenhang eine bivalente verzweigte oder unverzweigte C₃-C₁₂-Alkylen- oder Alkenylenkette, die den Silan-, Oligosiloxan- oder Polysiloxanteil mit dem Triazinrest verknüpft.

Beispiele für eine C₃-C₁₂-Alkylenkette sind Propylen, 2-Methylpropylen, Butylen, Pentylen und Hexylen.

Beispiele für eine C₃-C₁₂-Alkenylenkette sind 2-Propen-2-ylen, 2-Methyl-3-propenylen, 3-Buten-3-ylen und 4-Penten-4-ylen.

Bevorzugte Spacer sind -(CH₂)₃-, -(CH₂)₄-, -(CH₂)₅-, -[CH(CH₃)]-(CH₂)-, -(CH₂)₂-CH=CH-, -C(=CH₂)-CH₂-, -C(=CH₂)-(CH₂)₂-O-(CH₂)₄-, -(CH₂)₄-O-(CH₂)₂-.

Der Begriff Silane steht in diesem Zusammenhang für einen Rest SiR¹⁵R¹⁶R¹⁷, in der R¹⁵, R¹⁶, R¹⁷ unabhängig voneinander für C₁-C₆-Alkyl, C₁-C₆-Alkoxy oder Phenyl stehen.

Als Beispiele seien zu nennen: Si(CH₂-CH₃)₃, Si(CH₂-CH₂-CH₃)₃, Si(isopropyl)₃, Si(ter.butyl)₃, Si(tert.butyl)(CH₃)₂, Si(CH₃)₂-(hexyl), Si(OCH₃)₃, Si(OEt)₃, SiPh₃.

Der Begriff Oligosiloxane bedeutet einen Rest aus der Gruppe der allgemeinen Formel, bestehend aus SiR¹⁸ₘ(OSiR¹⁸₃)ₙ mit m=0, 1 oder 2; n=3, 2 oder 1 und m+n=3, R¹⁸-(Si(R¹⁸)₂-O-]ᵣ-Si(R¹⁸)₂-A und R¹⁸-[Si(R¹⁸)₂-O-]ᵣ-Si(A) (R¹⁸)-O-Si(R¹⁸)₃, in denen A eine chemische Bindung oder einen Spacer und R¹⁸ einen C₁-C₆-Alkylrest oder Phenylrest bedeutet und r für Werte von 1 bis 9 steht.

Der Begriff Polysiloxane beinhaltet beispielsweise einen Rest aus der Gruppe der allgemeinen Formel, bestehend aus A-[Si(R¹⁹)₂-O]ₛ-Si(R¹⁹)₂-A oder (R¹⁹)₃-Si-[O-Si(R¹⁹)₂]]ₜ-[O-Si(R¹⁹)(A)]_{q}-O-Si(R¹⁹)₃, in denen A eine chemische Bindung oder einen Spacer und R¹⁹ einen C₁-C₆-Alkylrest oder Phenylrest bedeutet, s und t für Werte von 4 bis 250 und q für Werte von 1 bis 30 stehen.

Beispiele für Silanyl-Triazine, bei denen R¹² einen Rest der Formel Sp-Sil darstellt finden sich in EP-A-0 933 376.

Im Vordergrund stehen Triazinverbindungen der Formel Ib, 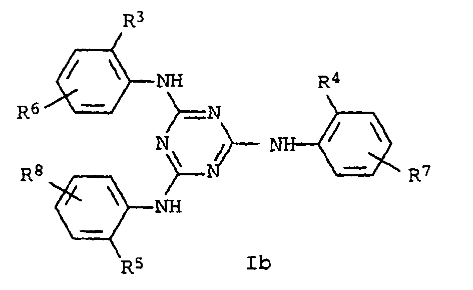 worin R³ bis R⁵ in ortho-Stellung zum Phenylaminorest des Triazins stehen.

Bevorzugt für die Verwendung als Lichtschutzmittel sind Trockenpulver, enthaltend mindestens ein 1,3,5-Triazinderivat der Formel Ib, in der die Substituenten unabhängig voneinander folgende Bedeutung haben:
- R³ bis R⁵: Wasserstoff, OH;
- R⁶ bis R⁸: C₁-C₁₂-Alkoxy, -C(=O)-X-R¹²;
- X: O, NR¹⁴;
- R¹² und R¹⁴: Wasserstoff, C₄-C₈-Alkyl.

Ein besonders vorteilhafter UVB-Filter ist 2,4,6-Trianilino-p-(carbo-2'-ethyl-hexyl-1'-oxi)-1,3,5-triazin, der von der BASF Aktiengesellschaft unter der Warenbezeichnung Uvinul^{®} T150 vermarktet wird.

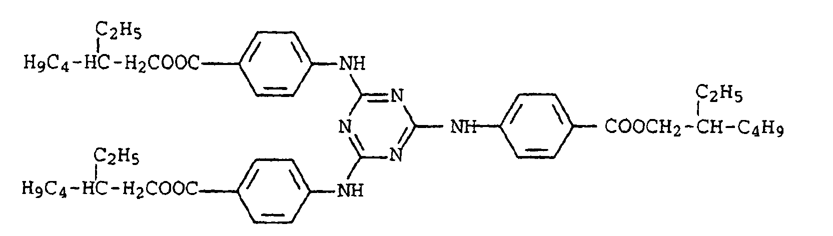

Uvinul^{®} T150 zeichnet sich durch gute UV-Absorptionseigenschaften mit einem außergewöhnlich hohen Extinktionskoeffizienten > 1500 bei 314 nm aus.

Als weitere schwer wasserlösliche oder wasserunlösliche organische UV-Filtersubstanzen aus der Gruppe der Triazine sind u.a. die folgenden, in WO 94/05645 und EP-A-0 444 323 beschriebenen Verbindungen zu nennen:

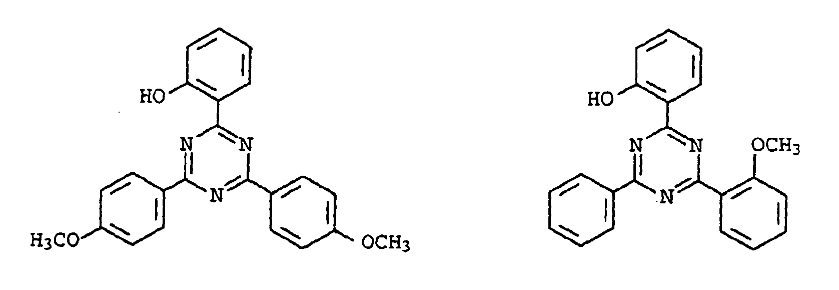

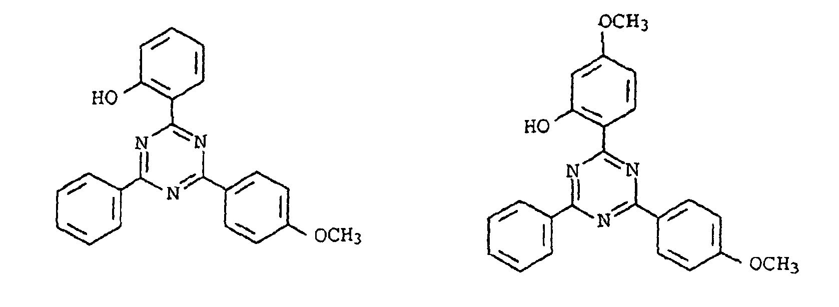

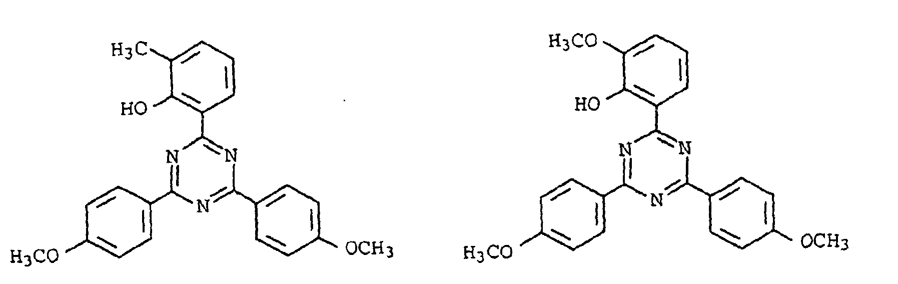

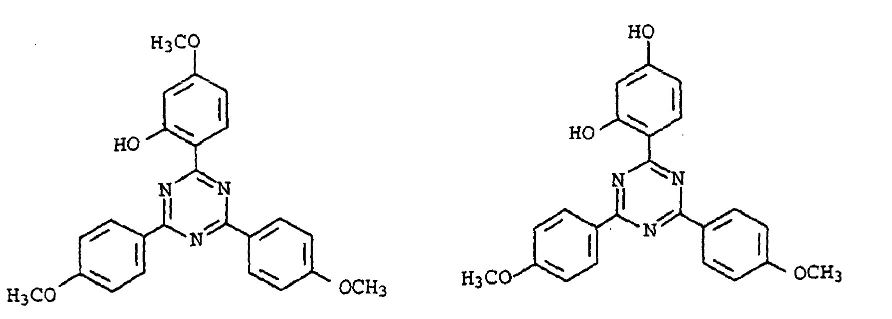

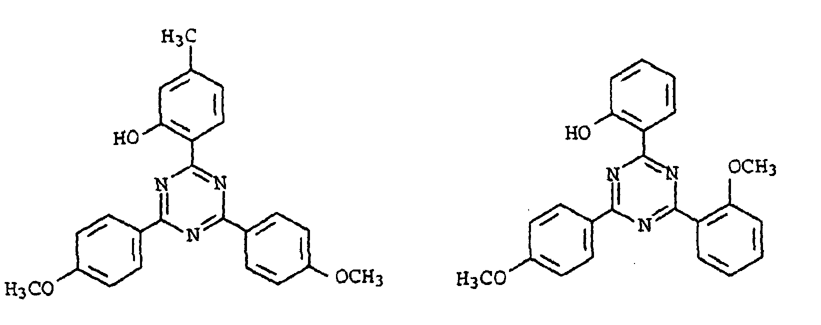

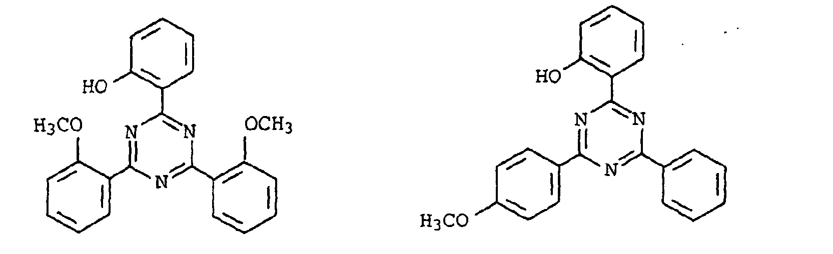

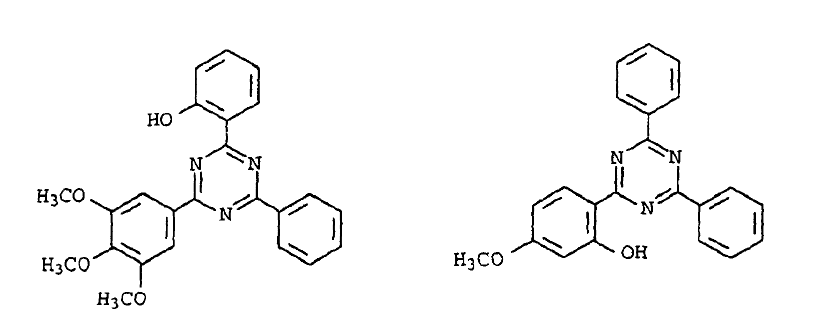

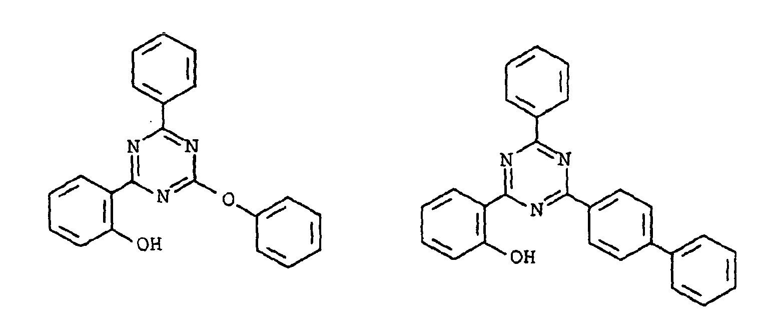

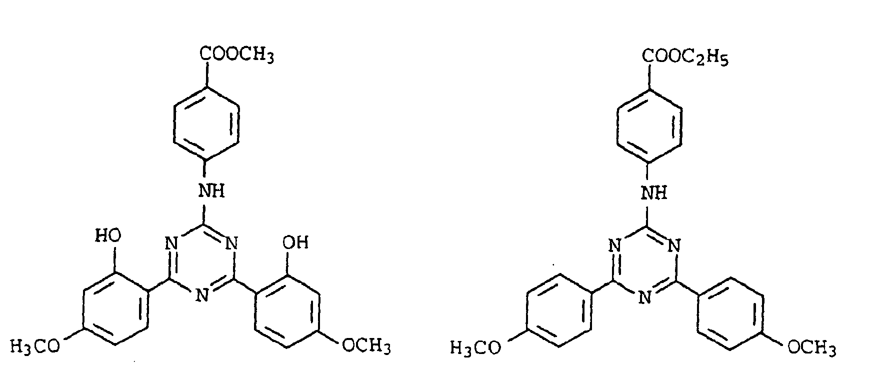

Bevorzugte Anilide sind Verbindungen der Formel II, in der W¹ und W² unabhängig voneinander C₁-C₁₈-Alkyl oder C₁-C₁₈-Alkoxy bedeuten.

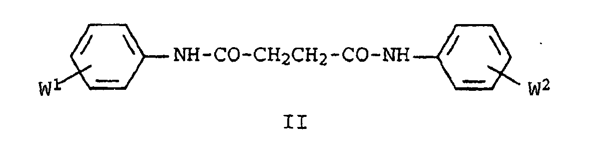

Besonders bevorzugt ist N-(2-ethoxyphenyl)-N'-(2-ethylphenyl)-ethandiamid.

Bevorzugte Triazole sind Verbindungen der Formel III, in der unabhängig voneinander T¹ C₁-C₁₈-Alkyl oder Wasserstoff und T² und T³ C₁-C₁₈-Alkyl bedeuten.

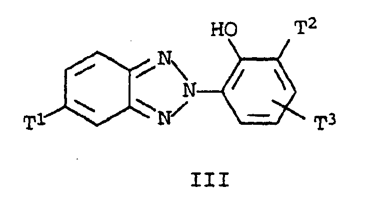

Eine weitere bevorzugte Verbindungsklasse wasserunlöslicher Triazole sind Verbindungen der Formel IIIa, in der T⁴ und T⁵ unabhängig voneinander C₁-C₁₈-Alkyl bedeuten.

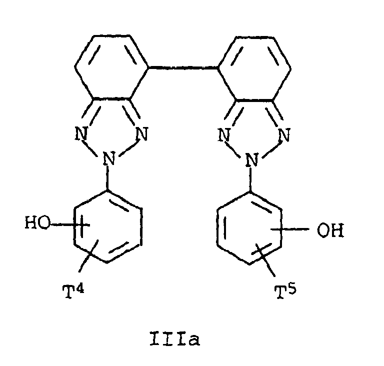

Ebenfalls bevorzugte Vertreter aus der Gruppe wasserunlöslicher Triazole sind Verbindungen der Formel IIIb und IIIc, in der T⁶ und T⁷ unabhängig voneinander C₁-C₁₈-Alkyl, bevorzugt tert.-Butyl, -C(CH₃)₂-CH₂-C(CH₃)₃ oder 2-Ethylhexyl bedeuten. Bei der besonders bevorzugten Verbindung der Formel IIIa stehen beide Reste T⁶ und T⁷ für -C(CH₃)₂-CH₂-C(CH₃)₃. T⁸ in der Formel IIIc bedeutet ebenfalls C₁-C₁₈-Alkyl, bevorzugt Methyl.

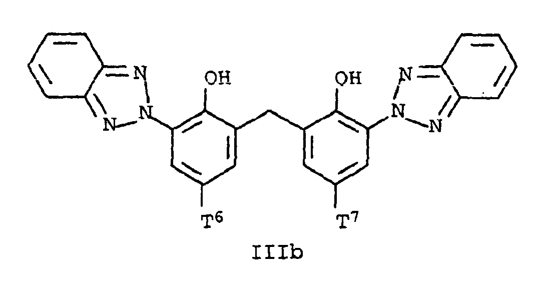

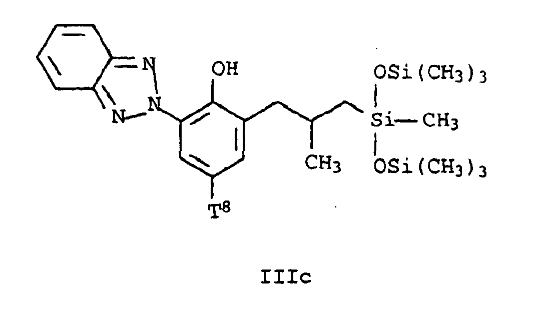

Bevorzugte Zimtsäureamide sind Verbindungen der Formel IV, in der unabhängig voneinander Y¹ und Y² Wasserstoff oder C₁-C₄-Alkyl, bevorzugt Methyl oder Ethyl bedeuten und Y³ für Aryl, bevorzugt Phenyl oder 4-Methoxyphenyl steht.

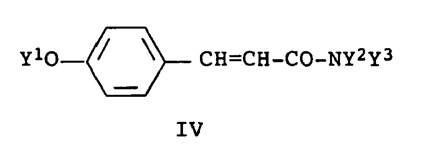

Bevorzugte sulfonierte Benzimidazole sind Verbindungen der Formel V, in der M für Wasserstoff, ein Alkalimetall - bevorzugt Natrium - oder ein Alkalimetall wie Magnesium, Calcium oder Zink steht.

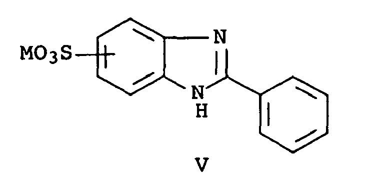

Bevorzugte Benzophenone sind Verbindungen der Formel VI, in der unabhängig voneinander M¹ bis M⁴ Wasserstoff oder C₁-C₄-Alkyl, M¹ und M⁴ bevorzugt Methyl oder Ethyl sowie M² und M³ bevorzugt Wasserstoff bedeuten.

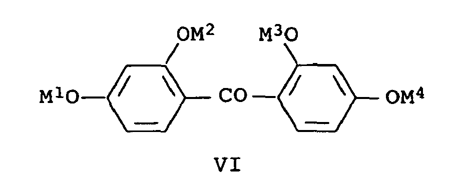

Als wasserunlösliche Wirkstoffe sind in diesem Zusammenhang solche Verbindungen gemeint, deren Wasserlöslichkeit bei 20°C kleiner 0,05 Gew.-%, bevorzugt kleiner 0,01 Gew.-%, ist.

Als schwer wasserlösliche Wirkstoffe sind solche Verbindungen gemeint, deren Wasserlöslichkeit bei 20°C kleiner 5 Gew.-%, bevorzugt kleiner 1 Gew.-%, besonders bevorzugt kleiner 0,5 Gew.-%, ganz besonders bevorzugt zwischen 0,5 und 0,05 Gew.-% liegt.

Als wasserlösliche Wirkstoffe sind in diesem Zusammenhang solche Verbindungen gemeint, deren Wasserlöslichkeit bei 20°C größer 5 Gew.-%, bevorzugt größer 10 Gew.-%, besonders bevorzugt größer 20 Gew.-%, ganz besonders bevorzugt größer 60 Gew.-% ist.

Bevorzugt ist ein Verfahren zur Herstellung fester Zubereitungen mindestens eines, für den Lebensmittel- und Tierfuttermittelbereich oder für pharmazeutische und kosmetische Anwendungen geeigneten, schwer wasserlöslichen oder wasserunlöslichen Wirkstoffs, dadurch gekennzeichnet, dass man im Verfahrensschritt a) mindestens einen der oben genannten schwer wasserlöslichen oder wasserunlöslichen Wirkstoffe in einer wässrigen molekulardispersen oder kolloiddispersen Lösung eines proteinhaltigen Schutzkolloids dispergiert.

Unter dem Begriff Dispergieren ist besonders bevorzugt die Herstellung wässriger Suspensionen sowie wässriger Emulsionen zu verstehen. Ganz besonders bevorzugt handelt es sich beim Dispergierschritt a) um die Herstellung einer Suspension mindestens eines der o.g. festen Wirkstoffe in einer wässrigen molekulardispersen oder kolloiddispersen Lösung eines proteinhaltigen Schutzkolloids, bei der die dispergierte Phase mindestens einen der Wirkstoffe als nanopartikuläre Teilchen enthält.

Als proteinhaltige Schutzkolloide eignen sich erfindungsgemäß sowohl wasserlösliche als auch wasserquellbare Proteine tierischen oder auch pflanzlichen Ursprungs. Als bevorzugte Schutzkolloide seien Casein, Caseinat, Rinder-, Schweine- oder Fischgelatine, insbesondere sauer oder basisch abgebaute Gelatine mit Bloom-Zahlen im Bereich von 0 bis 250, beispielsweise Gelatine A 100, A 200, B 100 und B 200 sowie niedermolekulare, enzymatisch abgebaute Gelatinetypen mit der Bloom-Zahl 0 und Molekulargewichten von 15000 bis 25000 D wie zum Beispiel Collagel A und Gelitasol P (Firma Stoess, Eberbach) sowie Mischungen dieser Gelatine-Sorten zu nennen. In manchen Fällen eignen sich aber auch Milchpulver, Vollmilch oder Magermilch als Schutzkolloide. Typische Vertreter für Pflanzenproteine sind Gluten, Zein, Soja- und Erbsenproteine. Besonders bevorzugte Schutzkolloide sind Casein und Caseinat.

Bezüglich näherer Einzelheiten zu den o.g. Schutzkolloiden wird auf R.A. Morton, Fat Soluble Vitamins, Intern. Encyclopedia of Food and Nutrition, Bd.9, Pergamon Press 1970, S. 128-131, ver iesen.

Die Wasserlöslichkeit bzw. Wasserquellbarkeit der o.g. Polymere ist dabei abhängig von der Temperatur, vom pH-Wert sowie von der Ionenstärke der Lösung.

Unter dem Begriff Ausflockung sind solche Vorgänge in einem kolloidalen System gemeint, welche eine Abscheidung der in diesem System dispergierten Teilchen in Form von Flocken und somit den Übergang Sol/Gel bewirken. Die Ausflockung kann in der Regel durch Zusatz von Flockungshilfsmittel, Elektrolyten, Polyelektrolyten, entgegengesetzt geladenen Kolloiden oder durch Erhitzen und damit durch Denaturierung des Proteins erfolgen. Eine vorteilhafte Methode zur Ausflockung des proteinhaltigen Schutzkolloids im Verfahrensschritt b) ist gekennzeichnet durch Einstellung des pH-Wertes der Dispersion auf einen Wert der im Bereich des isoelektrischen Punktes des als Schutzkolloid verwendeten Proteins liegt. Dieser Bereich umfasst erfindungsgemäß eine pH-Wert Einheit oberhalb und unterhalb des isoelektrischen Punktes, bevorzugt 0,5 pH-Wert Einheiten, besonders bevorzugt 0,1 bis 0,2 pH-Wert Einheiten. Ganz besonders bevorzugt wird die Flockung durch Einstellung des pH-Wertes der Dispersion auf einen Wert entsprechend dem isoelektrischen Punkt des als Schutzkolloid verwendeten Proteins ausgelöst.

Die Abtrennung des ausgeflockten Feststoffs vom Wasser und von gegebenenfalls zusätzlich verwendeten organischen Lösungsmitteln kann in an sich bekannter Weise, beispielsweise durch Filtration oder Zentrifugation erfolgen.

Die Überführung in ein Trockenpulver kann dabei u.a. durch Sprühtrocknung, Gefriertrocknung oder Trocknung im Wirbelbett, gegebenenfalls auch in Gegenwart eines Überzugsmaterials erfolgen. Als Überzugsmittel eignen sich u.a. Maisstärke, Kieselsäure oder auch Tricalciumphosphat.

Bei der Lyophilisation des abgetrennten Feststoffs können kryoprotektive Substanzen wie z.B. Trehalose oder Polyvinylpyrrolidone zugesetzt werden.

Eine weitere bevorzugte Ausführungsform des o.g. Verfahrens ist dadurch gekennzeichnet, dass man die im Verfahrensschritt a) hergestellte Suspension vor der Ausflockung mahlt. In diesem Fall wird der Wirkstoff vor dem Mahlvorgang bevorzugt in kristalliner Form suspendiert.

Die Mahlung kann dabei in an sich bekannter Weise z.B. mit einer Kugelmühle erfolgen. Dabei wird je nach verwendetem Mühlentyp so lange gemahlen, bis die Teilchen eine über Fraunhofer Beugung ermittelte mittlere Partikelgröße D[4,3] von 0,1 bis 100 µm, bevorzugt 0,2 bis 50 µm, besonders bevorzugt 0,5 bis 30 µm, ganz besonders bevorzugt 0,8 bis 20 µm, insbesondere 1,0 bis 10 µm aufweisen. Der Begriff D[4,3] bezeichnet den volumengewichteten mittleren Durchmesser (siehe Handbuch zu Malvern Mastersizer S, Malvern Instruments Ltd., UK).

Nähere Einzelheiten zur Mahlung und den dafür verwendeten Apparaturen finden sich u.a. in Ullmann's Encyclopedia of Industrial Chemistry, Sixth Edition, 1999, Electronic Release, Size Reduction, Kapitel 3.6.: Wet Grinding sowie in EP-A-0 498 824.

Eine ebenfalls bevorzugte Variante des erfindungsgemäßen Verfahrens ist dadurch gekennzeichnet, dass das Dispergieren in der Stufe a) folgende Schritte enthält:
a₁) Lösen eines oder mehrerer der oben genannten Wirkstoffe in einem mit Wasser mischbaren, organischen Lösungsmittel oder einer Mischung aus Wasser und einem mit Wasser mischbaren, organischen Lösungsmittel oder
a₂) Lösen eines oder mehrerer der oben genannten Wirkstoffe in einem mit Wasser nicht mischbaren, organischen Lösungsmittel und
a₃) Mischen der nach a₁) oder a₂) erhaltenen Lösung mit einer wässrigen molekulardispersen oder kolloiddispersen Lösung eines proteinhaltigen Schutzkolloids, wobei die hydrophobe Phase des Wirkstoffs als nanodisperse Phase entsteht.

Die in der Stufe a₁) verwendeten wassermischbaren Lösungsmittel sind vor allem wassermischbare, thermisch stabile, flüchtige, nur Kohlenstoff, Wasserstoff und Sauerstoff enthaltene Lösungsmittel wie Alkohole, Ether, Ester, Ketone und Acetale zu nennen. Zweckmäßig verwendet man solche Lösungsmittel, die mindestens zu 10 % wassermischbar sind, einen Siedepunkt unter 200°C aufweisen und/oder weniger als 10 Kohlenstoffe haben. Besonders bevorzugt werden Methanol, Ethanol, n-Propanol, Isopropanol, 1,2-Butandiol-1-methylether, 1,2-Propandiol-1-n-propylether, Tetrahydrofuran oder Aceton verwendet.

Der Begriff "ein mit Wasser nicht mischbares organisches Lösungsmittel" steht im Sinne der vorliegenden Erfindung für ein organisches Lösungsmittel mit einer Wasserlöslichkeit bei Normaldruck von weniger als 10 %. Als mögliche Lösungsmittel kommen dabei u.a. halogenierte aliphatische Kohlenwasserstoffe, wie z.B. Methylenchlorid, Chloroform und Tetrachlorkohlenstoff, Carbonsäureester wie Dimethylcarbonat, Diethylcarbonat, Propylencarbonat, Ethylformiat, Methyl-, Ethyl- oder Isopropylacetat sowie Ether wie Methyl-tert. butylether in Frage. Bevorzugte, mit Wasser nicht mischbare organische Lösungsmittel sind die folgenden Verbindungen aus der Gruppe, bestehend aus Dimethylcarbonat, Propylencarbonat, Ethylformiat, Ethylacetat, Isopropylacetat und Methyl-tert. butylether.

Bei der Verwendung eines mit Wasser nicht mischbaren Lösungsmittels gemäß Verfahrensschritt a₂) kann es von Vorteil sein, die nach Verfahrensschritt a₃) erhaltene Dispersion vor der Ausflockung des Proteins im Schritt b) von dem mit Wasser nicht mischbaren Lösungsmittel - beispielsweise durch Destillation - zu befreien.

Das erfindungsgemäße Verfahren ist ferner dadurch gekennzeichnet, dass es sich bevorzugt um die Herstellung Carotinoid-haltiger Trockenpulver handelt.

Als besonders bevorzugt sind erfindungsgemäß solche Carotinoid-haltigen Trockenpulver zu nennen, die die folgenden Wirkstoffe, ausgewählt aus der Gruppe, bestehend aus Astaxanthin, β-Carotin, β-Apo-8'-Carotinal, β-Apo-8'-Carotinsäureethylester, Canthaxanthin, Citranaxanthin, Echinenon, Lutein, Lycopin und Zeaxanthin enthalten.

Das für die o.g. Carotinoid-haltigen Trockenpulver ganz besonders bevorzugte Herstellverfahren ist dadurch gekennzeichnet, dass man
a) ein oder mehrere Carotinoide in einem mit Wasser mischbaren, organischen Lösungsmittel oder einer Mischung aus Wasser und einem mit Wasser mischbaren, organischen Lösungsmittel bei Temperaturen größer 30°C löst,
b) die erhaltene Lösung mit einer wässrigen Casein- oder Caseinat-Lösung mischt,
c) das Casein oder Caseinat zusammen mit dem Carotinoid bei einem pH-Wert der Dispersion ausflockt, der im Bereich von einer pH-Wert Einheit oberhalb und unterhalb des isoelektrischen Punktes von Casein oder Caseinat liegt,
d) den ausgeflockten Feststoff vom Wasser und vom Lösungsmittel abtrennt und trocknet.

Die Herstellung der o.g. Carotinoid-haltigen Zubereitungen erfolgt in der Regel so, dass man mindestens ein Carotinoid in einem mit Wasser mischbaren, organischen Lösungsmittel bei Temperaturen größer 30°C, vorzugsweise zwischen 50°C und 240°C, insbesondere 100°C bis 200°C, besonders bevorzugt 140°C bis 180°C, gegebenenfalls unter Druck, löst.

Da die Einwirkung hoher Temperaturen u.U. den gewünschten hohen all-trans Isomerenanteil herabsetzen kann, löst man das/die Carotinoid(e) möglichst rasch, beispielsweise im Sekundenbereich, z.B. in 0,1 bis 10 Sekunden, besonders bevorzugt in weniger als 1 Sekunde. Zur raschen Herstellung der molekulardispersen Lösung kann die Anwendung von erhöhtem Druck, z.B. im Bereich von 20 bar bis 80 bar, vorzugsweise 30 bis 60 bar, vorteilhaft sein.

Die so erhaltene molekulardisperse Lösung versetzt man anschließend direkt mit der gegebenenfalls gekühlten wässrigen molekulardispersen oder kolloiddispersen Lösung des Caseins oder Caseinats in der Weise, dass sich eine Mischungstemperatur von etwa 35°C bis 80°C einstellt.

Dabei wird die Lösungsmittelkomponente in die wässrige Phase überführt und die hydrophobe Phase des/der Carotinoid(e) entsteht als nanodisperse Phase.

Hinsichtlich einer näheren Verfahrens- und Apparatebeschreibung zur oben genannten Dispergierung wird an dieser Stelle auf EP-B-0 065 193 Bezug genommen.

Die im Verfahrensschritt c) durchgeführte Ausflockung des Caseins oder Caseinats erfolgt insbesondere bei einem pH-Wert der Dispersion im Bereich von 4,0 bis 5,5, bevorzugt im Bereich von 4,4 bis 5,2, besonders bevorzugt im Bereich von 4,6 bis 5,0, ganz besonders bevorzugt im Bereich von 4,7 bis 4,9. Am meisten bevorzugt ist die Ausflockung des Caseins oder Caseinats bei pH 4,8.

Als vorteilhafte Schutzkolloide werden nieder- und/oder hochmolekulares Casein oder Caseinat oder Mischungen davon verwendet. Bevorzugt verwendet man Na-Caseinat mit einem Molekulargewicht von 10000 bis 100000, besonders bevorzugt mit einem MW von 20000 bis 60000, beispielsweise Na-Caseinat der Fa. Lacto Bretagne Associés S.A. (Frankreich) mit einem MW von ca. 38000.

Einzelheiten zum eingesetzten Casein/Caseinat finden sich u.a. in Ullmann's Encyclopedia of Industrial Chemistry, 6^{th} Edition, 1998 Electronic Release, Chapter 11.1., Wiley-VCH, Weinheim, Germany sowie in CD Römpp Chemie Lexikon-Version 1.0, Stuttgart/New York: Georg Thieme Verlag 1995 und der darin zitierten Literatur.

Zur Erhöhung der mechanischen Stabilität des Endproduktes kann es in einigen Fällen zweckmäßig sein, dem Kolloid einen Weichmacher zuzusetzen, wie Zucker oder Zuckeralkohole, z.B. Saccharose, Glucose, Lactose, Invertzucker, Sorbit, Mannit oder Glycerin.

Zur Erhöhung der Stabilität des Wirkstoffes gegen oxidativen Abbau ist es vorteilhaft, Stabilisatoren wie α-Tocopherol, t-Butylhydroxytoluol, t-Butylhydroxyanisol, Ascorbinsäure oder Ethoxyquin zuzusetzen. Sie können entweder der wässrigen oder der Lösungsmittel-Phase zugesetzt werden, vorzugsweise werden sie jedoch gemeinsam mit den Wirkstoffen in der Lösungsmittel-Phase gelöst.

Unter Umständen kann es auch vorteilhaft sein, zusätzlich in der Lösungsmittel-Phase ein physiologisch zugelassenes Öl wie beispielsweise Sesamöl, Maiskeimöl, Baumwollsaatöl, Sojabohnenöl oder Erdnussöl sowie Ester mittelkettiger pflanzlicher Fettsäuren in einer Konzentration von 0 bis 500 Gew.-%, vorzugsweise 10 bis 300 Gew.-%, besonders bevorzugt 20 bis 100 Gew.-%, bezogen auf den/die Wirkstoff(e), zu lösen, das dann gemeinsam mit den Wirkstoffen und den genannten Zusatzstoffen beim Mischen mit der wässrigen Phase extrem feinteilig ausgefällt wird.

Die Erfindung betrifft auch feste Zubereitungen mindestens eines der oben genannten, für den Lebensmittel- und Tierfuttermittelbereich oder für pharmazeutische und kosmetische Anwendungen geeigneten, wasserlöslichen, schwer wasserlöslichen oder wasserunlöslichen Wirkstoffe, erhältlich nach einem der eingangs genannten Verfahren.

Bevorzugt sind dabei feste Zubereitungen, enthaltend mindestens einen, für den Lebensmittel- und Tierfuttermittelbereich oder für pharmazeutische und kosmetische Anwendungen geeigneten, schwer wasserlöslichen oder wasserunlöslichen Wirkstoff.

Der Wirkstoffgehalt in den erfindungsgemäßen Trockenpulvern liegt im Bereich von 0,1 bis 80 Gew.-%, bevorzugt 1,0 bis 75 Gew.-%, besonders bevorzugt 5,0 bis 70 Gew.-%, ganz besonders bevorzugt im Bereich von 20 bis 65 Gew.-%.

Als die bevorzugten festen Zubereitungen sind in diesem Zusammenhang Carotinoid-haltige Trockenpulver zu nennen, insbesondere solche Trockenpulver, enthaltend Carotinoide, ausgewählt aus der Gruppe, bestehend aus Astaxanthin, β-Carotin, β-Apo-8'-Carotinal, β-Apo-8'-Carotinsäureethylester, Canthaxanthin, Citranaxanthin, Echinenon, Lutein, Lycopin und Zeaxanthin.

In wässrigen Systemen - außerhalb des pH-Wertes des isoelektrischen Punktes des verwendeten proteinhaltigen Schutzkolloids - lassen sich die erfindungsgemäßen festen Zubereitungen wieder problemlos redispergieren.

Die erfindungsgemäßen Trockenpulver eignen sich insbesondere als Zusatz zu Lebens- und Tierfuttermitteln sowie als Zusatz zu kosmetischen und pharmazeutischen Zubereitungen. Typische Einsatzgebiete für die Carotinoid-haltigen Trockenpulver im Tierfuttermittelbereich sind beispielsweise die Fischpigmentierung in der Aquakultur sowie die Eidotter- und Broilerhautpigmentierung in der Geflügelzucht.

Für die oben genannte Verwendung werden die Trockenpulver vorteilhafterweise in Form öliger Suspensionen eingesetzt.

Gegenstand der vorliegenden Erfindung sind daher auch ölige Suspensionen, enthaltend als disperse Phase feste Zubereitungen mindestens eines, für den Lebensmittel- und Tierfuttermittelbereich oder für pharmazeutische und kosmetische Anwendungen geeigneten, wasserlöslichen, schwer wasserlöslichen oder wasserunlöslichen Wirkstoffs ausgewählt aus der Gruppe bestehend aus fettlöslichen Vitaminen, mehrfach ungesättigten Fettsäuren, Carotinoiden und organischen UV-Filtersubstanzen, die erhältlich sind durch
a) Lösen oder Dispergieren mindestens eines der oben genannten Wirkstoffe in einer wässrigen molekulardispersen oder kolloiddispersen Lösung eines proteinhaltigen Schutzkolloids,
b) Ausflockung des proteinhaltigen Schutzkolloids zusammen mit dem Wirkstoff aus der Dispersion durch Einstellung des pH-Wertes der Dispersion auf einen Wert, der im Bereich von einer pH-Wert Einheit oberhalb und unterhalb des isoelektrischen Punktes des als Schutzkolloid verwendeten Protein liegt
   und
c) Abtrennung des ausgeflockten Feststoffs vom Wasser und von gegebenenfalls zusätzlich verwendeten Lösungsmitteln und anschließendes Überführen in ein Trockenpulver,
wobei die Suspension einen Wassergehalt von 0,2 bis 4 Gew.-% aufweisen.

Bevorzugt sind außerdem solche öligen Suspensionen, die als Wirkstoff mindestens ein Carotinoid enthalten.

Die Carotinoide, die im Rahmen der Erfindung eingesetzt werden können, sind die bekannten, aus natürlichen Quellen oder synthetisch zugänglichen Vertreter dieser Stoffklasse. Darunter sind z.B. folgende Verbindungen zu verstehen: β-Carotin, Lycopin, Lutein, Astaxanthin, Zeaxanthin, Cryptoxanthin, Citranaxanthin, Canthaxanthin, Echinenon, Bixin, β-Apo-4-carotinal, β-Apo-8-carotinal, β-Apo-8-carotinsäureester, einzeln oder als Mischung. Bevorzugt sind mindestens ein Carotinoid aus der Gruppe, bestehend aus Astaxanthin, β-Carotin, β-Apo-8'-Carotinal, β-Apo-8'-Carotinsäureethylester, Canthaxanthin, Citranaxanthin, Echinenon, Lutein, Lycopin und Zeaxanthin. Als besonders bevorzugte Carotinoide sind Astaxanthin und Canthaxanthin zu nennen, ganz besonders bevorzugt wird Astaxanthin verwendet.

Als Schutzkolloide kann die disperse Phase neben den bereits genannten proteinhaltigen Verbindungen - beispielsweise Gelatine wie Rinder-, Schweine- oder Fischgelatine, Gelatinehydrolysate, Casein, Caseinat, Molkeprotein und Pflanzenproteine auch Stärke, Dextrin, Pektin, Gummi-Arabikum, modifizierte Stärke wie z.B. Na-Octenyl-succinat-Stärke, hochamylasehaltige Stärke (wie z.B. Hylon^{®}, Fa. National Starch), einzeln oder als Mischungen enthalten. Typische Vertreter für Pflanzenproteine sind Gluten, Zein, Soja-, Reis-, Kartoffel- und Erbsenproteine. Es können aber auch Polyvinylalkohol, Polyvinylpyrrolidon, Methylcellulose, Carboxymethylcellulose, Hydroxypropylcellulose und Alginate eingesetzt werden. Als bevorzugte Schutzkolloide seien mindestens ein proteinhaltiges Schutzkolloid aus der Gruppe, bestehend aus Casein, Caseinat, Sojaprotein, Sojaproteinhydrolysate, Schweine- und Fischgelatine oder eine modifizierte Stärke genannt.

Bezüglich näherer Einzelheiten zu den o.g. Schutzkolloiden wird auf R.A. Morton, Fat Soluble Vitamins, Intern. Encyclopedia of Food and Nutrition, Bd.9, Pergamon Press 1970, S. 128-131, verwiesen.

Die Schutzkolloide können zur Verbesserung ihrer Eigenschaften mit Emulgatoren kombiniert werden.

Die erfindungsgemäßen öligen Formulierungen, insbesondere die Carotinoid-haltigen öligen Suspensionen zeichnen sich u.a. auch dadurch aus, dass sie einen Wassergehalt bevorzugt von 0,5 bis 3 Gew.-% aufweisen.

Die mittlere Partikelgröße D[4,3] der dispersen Phase der öligen Suspensionen liegt im Bereich von 0,1 bis 100 µm, bevorzugt 0,2 bis 50 µm, besonders bevorzugt 0,5 bis 30 µm, ganz besonders bevorzugt 0,8 bis 20 µm, insbesondere im Bereich von 1,0 bis 10 µm. Der Begriff D[4,3] bezeichnet in diesem Zusammenhang den volumengewichteten mittleren Durchmesser (siehe Handbuch zu Malvern Mastersizer S, Malvern Instruments Ltd., UK).

Die bevorzugten Carotinoid-haltigen öligen Suspensionen sind ferner dadurch gekennzeichnet, dass mindestens ein Carotinoid in amorpher oder teilamorpher Form vorliegt. Die anhand von Röntgenbeugungsdiagrammen ermittelten amorphen Anteile der Carotinoide liegen im Bereich von 30 bis 100 %, bevorzugt im Bereich von 40 bis 99 %, besonders bevorzugt von 60 bis 98 %, ganz besonders bevorzugt im Bereich von 70 bis 95 %.

Durch den hohen Amorphizitätsgrad der Wirkstoffe, speziell der Carotinoide, in den öligen Suspensionen zeigen diese Formulierungen eine besonders hohe Bioverfügbarkeit, verknüpft mit einer sehr guten Farbausbeute bei der Pigmentierung beispielsweise von Eidottern oder Fischen wie z.B. von Lachsen.

Der Gehalt an Wirkstoffen in den öligen Suspensionen liegt im allgemeinen zwischen 0,1 und 50 Gew.-%, bevorzugt zwischen 0,2 und 30 Gew.-%, besonders bevorzugt zwischen 0,5 und 20 Gew.-%, ganz besonders bevorzugt zwischen 1,0 und 15 Gew.-%, bezogen auf die Gesamtmenge der öligen Suspension.

Das verwendete Dispersionsmittel kann bei 20°C fest oder flüssig sein und kann sowohl synthetischen, mineralischen, pflanzlichen als auch tierischen Ursprungs sein. Typische Vertreter sind u.a. Sesamöl, Maiskeimöl, Baumwollsaatöl, Sojabohnenöl oder Erdnussöl, Ester mittelkettiger pflanzlicher Fettsäuren, Speisetalg, Oleostearin sowie Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Bevorzugt zu nennen sind in diesem Zusammenhang essbare, bei 20°C flüssige Öle wie Sonnenblumenöl, Palmöl, Sesamöl, Maiskeimöl, Baumwollsaatöl, Sojabohnenöl oder Erdnussöl, Ester mittelkettiger Triglyceride sowie außerdem Fischöle wie beispielsweise Makrelen-, Sprotten- oder Lachsöl. Für die Tierernährung besonders bevorzugt sind Fischöle, Maiskeimöl, Sonnenblumenöl, Sojaöl und Erdnussöl. Für den Food-/Pharmabereich zusätzlich von Vorteil sind die Ester mittelkettiger Triglyceride.

Je nach verwendetem Dispersionsmittel (Öl oder Hartfett) können die erfindungsgemäßen Suspensionen sowohl in fließfähiger Form als fest/flüssig-System als auch in Abhängigkeit von ihrer Viskosität und vom Schmelzpunkt des Dispersionsmittels in fester Form, d.h. als heterogenes fest/fest-Stoffsystem vorliegen.

Zur Vermeidung von Sedimentationen der Carotinoid-haltigen Teilchen in den öligen Zubereitungen - beispielsweise bei längerer Lagerung - sind in einigen Fällen auch die bereits oben genannten Hartfette (z.B. Speisetalg oder Oleostearin) als Dispersionsmittel bevorzugt.

Die Menge des Dispersionsmittels beträgt im allgemeinen 20 bis 99,9 Gew.-%, bevorzugt 50 bis 99 Gew.-%, besonders bevorzugt 55 bis 97 Gew.-%, ganz besonders bevorzugt 60 bis 99 Gew.-%, bezogen auf die Gesamtmasse der öligen Suspension.

In manchen Fällen kann es erforderlich sein, dass die öligen Suspensionen außerdem Hilfsstoffe, wie z.B. Verdicker, Hartfette, Chelatbildner, wie z.B. Alkali- oder Erdalkalisalze der Citronensäure, Phytinsäure oder Phosphorsäure enthalten und/oder Emulgatoren enthalten.

Als Emulgatoren bzw. Solubilisatoren können beispielsweise Ascorbylpalmitat, Polyglycerin-Fettsäureester, Sorbitan-Fettsäureester, Propylenglycol-Fettsäureester oder Lecithin verwendet werden.

Im Falle der öligen Suspensionen, die als disperse Phase die oben beschriebenen ausgeflockten Feststoffe enthalten, beträgt die Menge des Dispersionsmittels im allgemeinen 50 bis 99,9 Gew.-%, bevorzugt 70 bis 99,8 Gew.-%, besonders bevorzugt 80 bis 99,5 Gew.-%, ganz besonders bevorzugt 90 bis 99 Gew.-%, bezogen auf die Gesamtmasse der öligen Dispersion.

Gegenstand der Erfindung ist auch ein Verfahren zur Herstellung Carotinoid-haltiger öliger Suspensionen, bevorzugt öliger Suspensionen, enthaltend als Wirkstoff mindestens ein Carotinoid aus der Gruppe, bestehend aus Astaxanthin, β-Carotin, β-Apo-8'-Carotinal, β-Apo-8'-Carotinsäureethylester, Canthaxanthin, Citranaxanthin, Echinenon, Lutein, Lycopin und Zeaxanthin, dadurch gekennzeichnet, dass man
a) ein Trockenpulver, enthaltend mindestens ein von einem oder mehreren Schutzkolloiden umhülltes Carotinoid in mindestens einem Öl bis auf eine mittlere Partikelgröße von 0,1 bis 100 µm mahlt oder
b) ein Trockenpulver, enthaltend mindestens ein von einem oder mehreren Schutzkolloiden umhülltes Carotinoid ohne Verwendung einer kontinuierlichen Phase bis auf eine mittlere Partikelgröße von 0,1 bis 100 µm mahlt und die gemahlenen Partikel anschließend in mindestens einem Öl suspendiert oder
c) eine Carotinoid-haltige Suspension, enthaltend als feste, disperse Phase mindestens ein von einem oder mehreren Schutzkolloiden umhülltes Carotinoid und als Dispersionsmittel Wasser oder ein Gemisch aus Wasser und einem wassermischbaren Lösungsmittel, bis auf eine mittlere Partikelgröße von 0,1 bis 100 µm mahlt, die feste Phase anschließend vom Wasser bzw. vom Wasser/Lösungsmittel-Gemisch befreit und die so erhaltenen gemahlenen Partikel in mindestens einem Öl suspendiert.

Die Mahlung kann in allen drei Verfahrensvarianten in an sich bekannter Weise z.B. mit einer Kugelmühle erfolgen. Dabei wird je nach verwendetem Mühlentyp so lange gemahlen, bis die Teilchen eine über Fraunhofer Beugung ermittelte mittlere Partikelgröße D[4,3] von 0,1 bis 100 µm, bevorzugt 0,2 bis 50 µm, besonders bevorzugt 0,5 bis 30 µm, ganz besonders bevorzugt 0,8 bis 20 µm, insbesondere 1,0 bis 10 µm aufweisen.

Nähere Einzelheiten zur Mahlung und den dafür eingesetzten Apparaturen finden sich u.a. in Ullmann's Encyclopedia of Industrial Chemistry, Sixth Edition, 1999, Electronic Release, Size Reduction, Kapitel 3.6.: Wet Grinding.

Die in der Verfahrensvariante c) verwendeten wassermischbaren Lösungsmittel sind vor allem wassermischbare, thermisch stabile, flüchtige, nur Kohlenstoff, Wasserstoff und Sauerstoff enthaltene Lösungsmittel wie Alkohole, Ether, Ester, Ketone und Acetale zu nennen. Zweckmäßig verwendet man solche Lösungsmittel, die mindestens zu 10 % wassermischbar sind, einen Siedepunkt unter 200°C aufweisen und/oder weniger als 10 Kohlenstoffe haben. Besonders bevorzugt werden Methanol, Ethanol, n-Propanol, Isopropanol, 1,2-Butandiol-1-methylether, 1,2-Propandiol-1-n-propylether, Tetrahydrofuran oder Aceton verwendet.

Die Abtrennung des Wassers bzw. Wasser/Lösungsmittelgemisches im Verfahren c) kann in an sich bekannter Weise, beispielsweise durch Destillation, gegebenenfalls unter vermindertem Druck erfolgen. Die Destillation wird in der Regel nach der Mahlung durchgeführt, sie kann aber auch schon im Verlauf des Mahlprozesses erfolgen.

Das erfindungsgemäße Verfahren ist ferner dadurch gekennzeichnet, dass es sich bei dem als Dispersionsmittel verwendeten Öl um ein bei 20°C flüssiges, essbares Öl oder um ein bei 20°C festes Hartfett handelt.

Nähere Einzelheiten zu den verwendeten Schutzkolloiden und Dispersionsmitteln finden sich in der bereits eingangs erfolgten Beschreibung zu den öligen Suspension.

Als Trockenpulver für die Mahlung können in der Regel alle aus dem Stand der Technik bekannten, festen Zubereitungen verwendet werden, in denen mindestens ein von einem Schutzkolloid umhülltes Carotinoid enthalten ist.

Bevorzugt sind die gemäß EP-A-0 065 193, EP-A-0 832 569, DE-A-199 19 751, WO 98/26008, EP-A-0 937 412 sowie in WO 91/062292 und WO 94/19411 hergestellten Trockenpulver zu nennen.

Die in EP-A-0 065 193 beschriebenen Carotinoid-Trockenpulver sind erhältlich nach einem Verfahren, das dadurch gekennzeichnet ist, dass man ein Carotinoid in einem mit Wasser mischbaren, organischen Lösungsmittel bei erhöhten Temperaturen kurzzeitig löst, aus der erhaltenen Lösung sofort durch schnelles Mischen mit einer wässrigen Lösung eines Schutzkolloids das Carotinoid in kolloid-disperser Form ausfällt und die erhaltene Dispersion in ein Trockenpulver überführt.

Gemäß EP-A-0 832 569 erhält man durch Tempern der nach EP-A-0 065 193 hergestellten Dispersion bei einer Temperatur zwischen 40°C und 90°C und anschließende Sprühtrocknung ein Carotinoid Trockenpulver, in dem die Wirkstoffpartikel weitgehend röntgenamorph vorliegen.

Die in DE-A-199 19 751 beschriebenen Trockenpulver enthalten mindestens ein Xanthophyll aus der Gruppe, bestehend aus Astaxanthin, Lutein und Zeaxanthin, das in einer Matrix aus Casein als Schutzkolloid eingebettet ist.

Die in WO 98/26008 beschriebenen Trockenpulver enthalten mindestens ein Xanthophyll, das in einer Matrix aus einem Gemisch nieder- und hochmolekularer Schutzkolloide eingebettet ist.

EP-A-0 937 412 betrifft pulverförmige Carotinoid-Zubereitungen, erhältlich durch a) Suspendieren eines Carotinoids in einem mit Wasser nicht mischbaren Lösungsmittel, gegebenenfalls in Gegenwart eines Antioxidans und/oder Öls; b) Erhitzen dieser Suspension auf eine Temperatur im Bereich von 100 bis 250°C; c) Mischen der nach b) erhaltenen Lösung mit einer wässrigen Schutzkolloid-Lösung bei einer Temperatur zwischen 20 und 100°C; d) Abtrennung des organischen Lösungsmittels und Überführung der Dispersion in ein Trockenpulver.

Die in WO 91/062292 offenbarten Trockenpulver sind erhältlich durch Mahlen der Carotinoide in einer wässrigen Schutzkolloid-lösung und anschließende Sprühtrocknung der gemahlenen wässrigen Carotnioid-Suspension.

In WO 94/19411 werden kristalline Carotinoide in Gegenwart einer wässrigen Schutzkolloid-Lösung gemahlen, durch kurzzeitiges Erhitzen bis zum Schmelzpunkt in eine amorphe Modifikation überführt und anschließend sprühgetrocknet.

Nähere Einzelheiten zur Herstellung dieser Trockenpulver finden sich in den Beschreibungen der o.g. Schriften.

Die für die Herstellung der erfindungsgemäßen öligen Suspensionen eingesetzten Carotinoid-Trockenpulver können neben den Schutzkolloiden weitere Hilfsstoffe wie Weichmacher, Emulgatoren und/oder Stabilisatoren enthalten.

Als Weichmacher verwendet man Zucker oder Zuckeralkohole, z.B. Saccharose, Glucose, Lactose, Invertzucker, Sorbit, Mannit oder Glycerin.

Das Verhältnis Schutzkolloid und Weichmacher zu Carotinoid wird im allgemeinen so gewählt, dass das Trockenpulver zwischen 0,5 und 65 Gew.-%, bevorzugt zwischen 1 und 25 Gew.-% Carotinoid, 10 bis 50 Gew.-%, bevorzugt 15 bis 35 Gew.-% eines Schutzkolloids, 20 bis 70 Gew.-%, bevorzugt 30 bis 60 Gew.-% eines Weichmachers, alle Prozentangaben bezogen auf die Trockenmasse des Pulvers, sowie gegebenenfalls untergeordnete Mengen eines Stabilisators enthält.

Zur Erhöhung der Stabilität des Carotinoide gegen oxidativen Abbau kann es vorteilhaft sein, 0 bis 10 Gew.-%, bevorzugt 0,5 bis 7,5 Gew.-%, bezogen auf die Gesamtmenge der Trockenpulver, eines oder mehrerer Stabilisatoren wie α-Tocopherol, t-Butylhydroxytoluol, t-Butylhydroxyanisol, Ascorbinsäure oder Ethoxyquin zuzusetzen.

Als Emulgatoren können beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.-%, vorzugsweise 5 bis 150 Gew.-%, besonders bevorzugt 10 bis 80 Gew.-%, bezogen auf die Carotinoide im Trockenpulver vorliegen.

Im Falle einer Mischung von Carotinoiden ist es bei dem erfindungsgemäßen Mahlverfahren möglich, alle in der Suspension verwendeten Komponenten als Gesamtmischung zu mahlen. Es kann aber auch jedes einzeln zu mahlende Carotinoid in hoher Konzentration in dem zu verwendenden Öl gemahlen werden. Die Endzubereitung ergibt sich dann durch eine Abmischung der jeweiligen Einzelsuspensionen.

Die erfindungsgemäßen öligen Suspensionen können vor der Anwendung durch Zusatz von Fetten oder Ölen auf die jeweilige Gebrauchskonzentration verdünnt werden. Die Verdünnung kann beispielsweise unter Rühren bei erhöhten Temperaturen wie z.B. 30 bis 80°C erfolgen.

Die oben genannten Suspensionen eignen sich u.a. als Zusatzstoff zu Tierfuttermitteln und Lebensmittelzubereitungen bzw. Mischfutter, als Mittel für die Herstellung pharmazeutischer und kosmetischer Zubereitungen sowie für die Herstellung von Nahrungsergänzungspräparaten im Human- und Tierbereich.

Bevorzugt lassen sich die Suspensionen als Futtermittelzusatz in der Tierernährung einsetzen, beispielsweise durch Einmischen in Futtermittelpellets bei der Extrusion oder zum Auftragen bzw. Aufsprühen auf Futtermittelpellets.

Die Anwendung als Futtermittelzusatzstoff erfolgt insbesondere durch direktes Aufsprühen der erfindungsgemäßen Suspensionen, gegebenenfalls nach Verdünnung mit Ölen, beispielsweise auf Tierfutterpellets als sogenannte "post-pelleting-application".

Eine bevorzugte Ausführungsform des Sprühverfahrens besteht darin, dass man die Futtermittelpellets unter vermindertem Druck mit der öligen Suspension belädt.

Beispiele hierfür finden sich u.a. in GB-A-2 232 573 sowie in EP-A-0 556 883.

Typische Einsatzgebiete im Lebensmittelbereich sind beispielsweise die Vitaminierung und Färbung von Getränken, Milchprodukten wie Joghurt, Milchmixgetränken oder Milchspeiseeis sowie von Puddingpulvern, Eiprodukten, Backmischungen und Süßwaren.

Im Kosmetikbereich können die öligen Suspensionen beispielsweise für dekorative Körperpflegemittel beispielsweise in Form einer Creme, einer Lotion, als Lippenstifte oder Make-up verwendet werden.

Gegenstand der Erfindung sind ferner Nahrungsergänzungsmittel, Tierfuttermittel, Lebensmittel sowie pharmazeutische und kosmetische Zubereitungen, enthaltend die eingangs beschriebenen öligen Suspensionen.

Bevorzugt richtet sich die Erfindung auf Tierfuttermittel, insbesondere auf Futtermittelpellets, die mit den Suspensionen beladen werden.

Unter Nahrungsergänzungspräparate sowie pharmazeutische Zubereitungen, die die erfindungsgemäße Suspension enthalten, sind u.a. Tabletten, Dragees sowie bevorzugt Hart- und Weichgelatinekapseln zu verstehen.

Kosmetische Zubereitungen, die die erfindungsgemäßen Suspensionen enthalten können, sind beispielsweise topisch anwendbare Zubereitungen, insbesondere dekorative Körperpflegemittel wie Lippenstifte, Gesichts-Make-up in Form einer Creme sowie Lotionen.

Im Falle der eingangs beschriebenen, UV-Licht absorbierenden Wirkstoffen (Lichtschutzmitteln) eignen sich die erfindungsgemäßen Lichtschutzmittel-haltigen festen Zubereitungen sowie die daraus hergestellten öligen Dispersionen auch als photostabile UV-Filter in kosmetischen und pharmazeutischen Zubereitungen zum Schutz der menschlichen Haut oder menschlicher Haare gegen Sonnenstrahlen aber auch gegen künstliches Licht, welches hohe UV-Anteile aufweist, allein oder zusammen mit für kosmetische oder pharmazeutische Zubereitungen bekannten, im UV-Bereich absorbierenden Verbindungen. Die kosmetischen und pharmazeutischen Zubereitungen als solche werden zugleich natürlich auch stabilisiert, um möglichst lange wirksam zu bleiben.

Demgemäß sind auch Gegenstand der vorliegenden Erfindung Lichtschutzmittel enthaltende kosmetische und pharmazeutische Zubereitungen zum Schutz der menschlichen Haut oder menschlicher Haare gegen UV-Licht im Bereich von 280 bis 400 nm, dadurch gekennzeichnet, dass sie in einem kosmetisch oder pharmazeutisch geeigneten Träger als photostabile UV-Filter wirksame Mengen einer Formulierung schwer wasserlöslicher oder wasserunlöslicher organischer UV-Filtersubstanzen - allein oder zusammen mit an sich für kosmetische und pharmazeutische Zubereitungen bekannten, im UV-A- und UV-B-Bereich absorbierenden Verbindungen - enthalten, wobei es sich bei den Formulierungen um die eingangs genannten erfindungsgemäßen festen Zubereitungen oder den daraus hergestellten öligen Dispersionen handelt.

Die Menge an schwer wasserlöslicher oder wasserunlöslicher organischer UV-Filtersubstanz in Form der erfindungsgemäßen Formulierungen, die in den kosmetischen und pharmazeutischen Zubereitungen eingesetzt wird, liegt im Bereich von 0,05 bis 20 Gew.-%, bevorzugt 0,1 bis 10 Gew.-%, besonders bevorzugt im Bereich von 1 bis 7 Gew.-%, bezogen auf die gesamte Menge der kosmetischen und pharmazeutischen Zubereitung.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wässriger Basis möglich. Demgemäß kommen Öle, Öl-in-Wasser- und Wasser-in-Öl-Emulsionen, Cremes und Pasten, Lippenschutzstiftmassen oder fettfreie Gele in Betracht.

Als Emulsionen kommen u.a. auch O/W-Makroemulsionen, O/W-Mikroemulsionen oder O/W/O-Emulsionen mit in dispergierter Form vorliegenden aminosubstituierten Hydroxybenzophenonen der Formel I in Frage, wobei die Emulsionen durch Phaseninversionstechnologie, gemäß DE-A-197 26 121 erhältlich sind.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkoimmission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäuure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis ∝mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Der Gesamtanteil der Hilfs- und Zusatzstoffe kann 1 bis 80, vorzugsweise 6 bis 40 Gew.-% und der nicht wässrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholischwässrige Lotionen.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit den erfindungsgemäß zu verwendenden Formulierungen angewandt werden, kommen beliebige UV-A- und UV-B-Filtersubstanzen in Betracht. Beispielsweise sind zu nennen:

| Nr. | Stoff | CAS-Nr. |
|---|---|---|
| | | (=Säure) |
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Sulfo)benzyliden-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxy-benzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4'-methoxy-dibenzoylmethan | 70356-09-1 |
| 29 | 2,2',4,4'-Tetrahydroxybenzophenon | 131-55-5 |
| 30 | 2,2`-Methylen-bis-[6(2H-benzotriazol-2-yl)-4-(1,1,3,3,-tetramethylbutyl)phenol] | 103597-45-1 |
| 31 | 2,2'-(1,4-Phenylen)-bis-1H-benzimidazol-4,6-disulfonsäure, Na-Salz | 180898-37-7 |
| 32 | 2,4-bis-[4-(2-Ethylhexyloxy)-2-hydroxy]phenyl-6-(4-methoxyphenyl)-(1,3,5)-triazin | 187393-00-6 |
| 33 | 3-(4-Methylbenzyliden)-campher | 36861-47-9 |
| 34 | 4-Bis(polyethoxy)paraaminobenzoesäurepolyethoxyethylester | 113010-52-9 |
| 35 | 2,4-Dihydroxybenzophenon | 131-56-6 |
| 36 | 2,2'-Dihydroxy-4,4'-dimethoxybenzophenon-5,5'-dinatriumsulfonat | 3121-60-6 |

Auch polymere oder polymergebundene Filtersubstanzen können erfindungsgemäß verwendet werden.

Die erfindungsgemäßen kosmetischen und dermatologischen Zubereitungen können vorteilhafterweise außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten. Besonders bevorzugt handelt es sich um Pigmente auf der Basis TiO₂ und ZnO.

Es ist besonders vorteilhaft im Sinne der vorliegenden Erfindung, wenngleich nicht zwingend, wenn die anorganischen Pigmente in hydrophober Form vorliegen, d.h. dass sie oberflächlich wasserabweisend behandelt sind. Diese Oberflächenbehandlung kann darin bestehen, dass die Pigmente nach an sich bekannter Weise, wie in DE-A-33 14 742 beschrieben, mit einer dünnen hydrophoben Schicht versehen sind.

Zum Schutz menschlicher Haare vor UV-Strahlen können die erfindungsgemäßen Lichtschutzmittel-Formulierungen in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Formulierungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Formulierungen hergestellten Emulsionen zeichnen sich besonders durch ihre hohe Stabilität, die Formulierungen I selber durch ihre hohe Photostabilität aus, und die damit hergestellten Zubereitungen durch ihr angenehmes Hautgefühl aus.

Die UV-Filterwirkung der erfindungsgemäßen Formulierungen kann auch zur Stabilisierung von Wirk- und Hilfsstoffen in kosmetischen und pharmazeutischen Formulierungen ausgenutzt werden.

Die erfindungsgemäßen Zubereitungen zeichnen sich durch ein besonders hohes Absorptionsvermögen im Bereich der UV-B-Strahlung mit scharfer Bandenstruktur und hohen Lichtschutzfaktoren aus.

Insbesondere der hohe Lichtschutzfaktor der Zubereitungen, der bereits bei niedrigen Konzentrationen an UV-absorbierenden Wirkstoffen gemessen wurde, war überraschend.

In dem nachfolgenden Beispiel wird die Herstellung der erfindungsgemäßen festen Zubereitungen sowie der öligen Suspensionen näher erläutert.

### Beispiel 1

### Astaxanthin-Trockenpulver

45 g kristallines Astaxanthin wurden bei Raumtemperatur in 375 g eines azeotropen Isopropanol/Wasser-Gemischs suspendiert. Diese Wirkstoffsuspension wurde dann auf 96°C erwärmt und bei einer Flussrate von 2,1 kg/h kontinuierlich mit weiterem Isopropanol/Wasser-Azeotrop der Temperatur 227 °C und einer Flussrate von 2,7 kg/h vermischt, wobei sich Astaxanthin bei einer Mischungstemperatur von 169°C und bei einem Druck von 60 bar auflöste. Die so erhaltene Wirkstofflösung wurde dann mit einer wässrigen Phase, bestehend aus einer Lösung von 80 g Na-Caseinat in 12 1 destilliertem Wasser, in der der pH-Wert mit 18 mL 1 M NaOH auf pH 8,0 eingestellt worden war, bei einer Flussrate von 56,1 kg/h vermischt.

Die bei der Mischung entstandenen nanopartikulären Wirkstoffteilchen wiesen im Isopropanol/Wasser-Gemisch eine Teilchengröße von 100 nm auf. Anschließend wurde die Wirkstoff-Dispersion mit 1 M HCl auf pH 4,8 eingestellt, so dass die Wirkstoff/Caseinat-Teilchen ausgeflockt wurden. Nach Filtration der ausgeflockten Teilchen über einen Filtersack und anschließender Gefriertrocknung erhielt man ein Trockenpulver mit einen Astaxanthin-Gehalt von 36 Gew.-%.

### Beispiel 2

### Astaxanthin-Trockenpulver

50 g kristallines Astaxanthin und 5,6 g Ethoxiquin wurden in 416 g eines azeotropen Isopropanol/Wasser-Gemischs bei Raumtemperatur suspendiert. Die Wirkstoffsuspension wurde dann auf 97°C erwärmt und bei einer Flussrate von 2,1 kg/h kontinuierlich mit weiterem Isopropanol/Wasser-Azeotrop der Temperatur von 216°C und einer Flussrate von 2,7 kg/h vermischt, wobei sich Astaxanthin bei einer Mischungstemperatur von 169°C bei einem Druck von 60 bar auflöste. Die Wirkstofflösung wurde anschließend mit einer wässrigen Phase, bestehend aus einer Lösung von 23,3 g Na-Caseinat in 14 l destilliertem Wasser, in dem der pH-Wert mit 5 mL 1 M NaOH auf pH 8,3 eingestellt worden war, bei einer Flussrate von 55,6 kg/h vermischt.

Die bei der Mischung entstandenen Wirkstoffteilchen wiesen im Isopropanol/Wasser-Gemisch eine Teilchengröße von 116 nm auf. Anschließend wurde diese Wirkstoff-Dispersion mit 1 M HCl auf pH 4,8 eingestellt, so dass die Wirkstoff/Caseinat-Teilchen ausgeflockt wurden. Danach wurde über einen Filtersack filtriert und der Filterkuchen getrocknet. Der erhaltene Feststoff wies einen Astaxanthin-Gehalt von 62 Gew.-% auf.

### Beispiel 3

### Lycopin-Trockenpulver

45 g kristallines Lycopin, 3,6 g Palmitinsäure und 6,6 g Tocopherol wurden bei Raumtemperatur in 388 g eines azeotropen Isopropanol/Wasser-Gemischs suspendiert. Die Wirkstoffsuspension wurde dann auf 94°C erwärmt und bei einer Flussrate von 2,0 kg/h kontinuierlich mit weiterem Isopropanol/Wasser-Azeotrop der Temperatur von 206°C und einer Flussrate von 3,3 kg/h vermischt, wobei sich Lycopin bei einer Mischungstemperatur von 171°C und bei einem Druck von 63 bar auflöste. Die so erhaltene Wirkstofflösung wurde mit einer wässrigen Phase, bestehend aus einer Lösung von 80 g Na-Caseinat in 7 l destilliertem Wasser, in dem der pH-Wert mit 19 mL 1 M NaOH auf pH 8,0 eingestellt worden war, bei einer Flussrate von 33,8 kg/h vermischt.

Die bei der Mischung entstandenen Wirkstoffteilchen wiesen im Isopropanol/Wasser-Gemisch eine Teilchengröße von 125 nm auf. Anschließend wurde die Wirkstoff-Dispersion mit 1 M HCl auf pH 4,8 eingestellt, so dass die Wirkstoff/Caseinat-Teilchen ausgeflockt wurden. Danach wurde über einen Filtersack filtriert und gefriergetrocknet. Der getrocknete Filterkuchen wies einen Lycopin-Gehalt von 32 Gew.-% auf.

### Beispiel 4

### β-Carotin-Trockenpulver

45 g β-Carotin, 3,6 g Ascorbylpalmitat und 6,6 g Tocopherol wurden in 388 g eines azeotropen Isopropanol/Wasser-Gemischs bei Raumtemperatur suspendiert. Die Wirkstoffsuspension wurde dann auf 96°C erwärmt und bei einer Flussrate von 2,1 kg/h kontinuierlich mit weiterem Isopropanol/Wasser-Azeotrop der Temperatur von 210°C und einer Flussrate von 3,0 kg/h vermischt, wobei sich β-Carotin bei einer Mischungstemperatur von 170°C bei einem Druck von 62 bar auflöste. Die Wirkstofflösung wurde dann mit einer wässrigen Phase, bestehend aus einer Lösung von 80 g Na-Caseinat in 7 l destilliertem Wasser, in dem der pH-Wert mit 18 mL 1 M NaOH auf pH 8,0 eingestellt worden war, bei einer Flussrate von 35,5 kg/h vermischt.

Die bei der Mischung entstandenen Wirkstoffteilchen wiesen im Isopropanol/Wasser-Gemisch eine Teilchengröße von 138 nm auf. Anschließend wurde diese Wirkstoff-Dispersion mit 1 M HCl auf pH 4,8 eingestellt, so dass die Wirkstoff/Caseinat-Teilchen ausgeflockt wurden. Danach wurde über einen Filtersack filtriert. Der Filterkuchen wurde anschließend über Gefriertrocknung getrocknet. Der getrocknete Filterkuchen wies einen β-Carotin-Gehalt von 32 Gew.-% auf.

### Beispiel 5

### Astaxanthin-Trockenpulver (Saure Fahrweise)

45 g kristallines Astaxanthin und 4,5 g Vanillin wurden in 375 g eines azeotropen Isopropanol/Wasser-Gemischs bei Raumtemperatur suspendiert. Die Wirkstoffsuspension wurde dann auf 98°C erwärmt und bei einer Flussrate von 2,1 kg/h kontinuierlich mit weiterem Isopropanol/Wasser-Azeotrop der Temperatur von 230°C und einer Flussrate von 2,8 kg/h vermischt, wobei sich Astaxanthin bei einer Mischungstemperatur von 171°C und bei einem Druck von 61 bar auflöste. Die Wirkstofflösung wurde sodann mit einer wässrigen Phase, bestehend aus einer Lösung von 80 g Na-Caseinat (Lacto Bretagne Associés) in 12 000 g destilliertem Wasser, in dem der pH-Wert mit 88 g 1 M HCl auf pH 2,9 eingestellt worden war, bei einer Flussrate von 55,2 kg/h vermischt.

Die bei der Mischung entstandenen Wirkstoffteilchen wiesen im Isopropanol/Wasser-Gemisch eine Teilchengröße von 1,2 µm auf. Anschließend wurde die Wirkstoff-Dispersion mit 1 M NaOH auf pH 4,8 eingestellt, so dass die Wirkstoff/Caseinat-Teilchen geflockt wurden. Danach wurde über einen Filtersack filtriert. Der Filterkuchen wurde anschließend über Gefriertrocknung getrocknet. Der getrocknete Filterkuchen wies einen Astaxanthin-Gehalt von 35 Gew.-% auf.

### Beispiel 6

### Mahlung von Lycopin mit Caseinat

3,3 g kristallines Lycopin, 2,5 g Na-Caseinat und 0,33 g Ascorbylpalmitat wurden in 40 g VE-Wasser bei Raumtemperatur suspendiert und der pH-Wert der Suspension mit 3 g 1 M NaOH alkalisch gestellt. Die Wirkstoff-Suspension wurde dann zusammen mit ca. 200 g Zirkonoxid-Keramikmahlperlen vom Durchmesser 1 mm in einer 100 ml-Glasflasche auf dem Red-Devil dispergiert. Nach einer Mahldauer von 3, 6 und 12 Stunden wurden Proben entnommen, um den Mahlfortschritt zu charakterisieren. Die mittleren Teilchengrößen zu diesen Zeiten betrugen 651 nm bei 67 % Varianz, 487 nm bei 50 % Varianz und 494 nm bei 55 % Varianz. Der pH-Wert der Endprobe betrug pH = 7,7 bei einem E1/1-Wert von 136.

Anschließend wurde diese Wirkstoff-Dispersion mit 1 M HCl auf pH 4,8 eingestellt, so dass die Wirkstoff/Caseinat-Teilchen ausgeflockt wurden. Nach Filtration über einen Filtersack wurde der Filterkuchen anschließend gefriergetrocknet.

### Beispiel 7

### Astaxanthin-Suspension in Öl

Zur Herstellung einer hochkonzentrierten öligen Astaxanthin-Suspension wurden 20 g des nach Beispiel 2 erhaltenen Astaxanthin-Trockenpulvers mit 1,0 g Ethoxiquin, 1,0 g Konservierungsmittel (BHT) und 4,0 g Emulgator (Span 65, Sigma) in 100 g Neutralöl (Delios SK, Grünau) mittels eines Ultra Turrax 5 Minuten lang suspendiert. Die erhaltene sedimentationsstabile Öl-Suspension hatte einen Astaxanthingehalt von 8,5 Gew.-%, bei einer Sekundär-Teilchengröße von 34 µm.

Nach der Abtrennung von den Mahlkörpern wurde ein Teil der Suspension mit der 10 fachen Menge des verwendeten Öls verdünnt und über 12 h stehengelassen. Weder die unverdünnte noch die verdünnte Suspension zeigten über diesen Zeitraum Sedimentationserscheinungen.

## Patentansprüche

1. Verfahren zur Herstellung fester Zubereitungen mindestens eines, für den Lebensmittel- und Tierfuttermittelbereich oder für pharmazeutische und kosmetische Anwendungen geeigneten, wasserlöslichen, schwer wasserlöslichen oder wasserunlöslichen Wirkstoffs, ausgewählt aus der Gruppe, bestehend aus fettlöslichen Vitaminen, wasserlöslichen Vitaminen, mehrfach ungesättigten Fettsäuren, Carotinoiden und organischen UV-Filtersubstanzen durch
a) Lösen oder Dispergieren mindestens eines der oben genannten Wirkstoffe in einer wäßrigen molekulardispersen oder kolloiddispersen Lösung eines proteinhaltigen Schutzkolloids,
b) Ausflockung des proteinhaltigen Schutzkolloids zusammen mit dem Wirkstoff aus der Dispersion
und
c) Abtrennung des ausgeflockten Feststoffs vom Wasser und von gegebenenfalls zusätzlich verwendeten Lösungsmitteln und anschließendes Überführen in ein Trockenpulver
**dadurch gekennzeichnet, dass** man im Verfahrensschritt b) die Flockung durch Einstellung des pH-Wertes der Dispersion auf einen Wert, der im Bereich von einer pH-Wert Einheit oberhalb und unterhalb des isoelektrischen Punktes des als Schutzkolloid verwendeten Proteins liegt auslöst.

2. Verfahren nach Anspruch 1 zur Herstellung fester Zubereitungen mindestens eines, für den Lebensmittel- und Tierfuttermittelbereich oder für pharmazeutische und kosmetische Anwendungen geeigneten, schwer wasserlöslichen oder wasserunlöslichen Wirkstoffs, **dadurch gekennzeichnet, dass** man im Verfahrensschritt a) mindestens einen der oben genannten Wirkstoffe in einer wäßrigen molekulardispersen oder kolloiddispersen Lösung eines proteinhaltigen Schutzkolloids dispergiert.

3. Verfahren nach Anspruch 2, **dadurch gekennzeichnet, dass** es sich beim Dispergierschritt a) um die Herstellung einer Suspension mindestens eines festen Wirkstoffs in einer wässrigen molekulardispersen oder kolloiddispersen Lösung eines proteinhaltigen Schutzkolloids handelt.

4. Verfahren nach Anspruch 3, **dadurch gekennzeichnet, dass** man die im Verfahrensschritt a) hergestellte Suspension vor der Ausflockung mahlt.

5. Verfahren nach Anspruch 2, **dadurch gekennzeichnet, dass** das Dispergieren in der Stufe a) folgende Schritte enthält:
a₁) Lösen eines oder mehrerer schwer wasserlöslicher oder wasserunlöslicher Wirkstoffe, ausgewählt aus der Gruppe, bestehend aus fettlöslichen Vitaminen, mehrfach ungesättigten, Carotinoiden und organischen UV-Filtersubstanzen in einem mit Wasser mischbaren, organischen Lösungsmittel oder einer Mischung aus Wasser und einem mit Wasser mischbaren, organischen Lösungsmittel oder
a₂) Lösen eines oder mehrerer schwer wasserlöslicher oder wasserunlöslicher Wirkstoffe, ausgewählt aus der Gruppe, bestehend aus fettlöslichen Vitaminen, mehrfach ungesättigten Fettsäuren, Carotinoiden und organischen UV-Filtersubstanzen in einem mit Wasser nicht mischbaren, organischen Lösungsmittel und
a₃) Mischen der nach a₁) oder a₂) erhaltenen Lösung mit einer wäßrigen molekulardispersen oder kolloiddispersen Lösung eines proteinhaltigen Schutzkolloids, wobei die hydrophobe Phase des Wirkstoffs als nanodisperse Phase entsteht.

6. Verfahren nach Anspruch 5, **dadurch gekennzeichnet, dass** man bei der Ausübung von Verfahrensschritt a₂) das mit Wasser nicht mischbare Lösungsmittel vor der Ausflockung des Schutzkolloids abdestilliert.

7. Verfahren nach einem der Ansprüche 1 bis 6, **dadurch gekennzeichnet, dass** man als Schutzkolloid Casein oder ein Caseinat verwendet.

8. Verfahren nach einem der Ansprüche 1 bis 7, **dadurch gekennzeichnet, dass** es sich dabei um die Herstellung Carotinoid-haltiger Trockenpulver handelt.

9. Verfahren nach Anspruch 8 zur Herstellung von Trockenpulvern, enthaltend Carotinoide, ausgewählt aus der Gruppe, bestehend aus Astaxanthin, β-Carotin, β-Apo-8'-Carotinal, β-Apo-8'-Carotinsäureethylester, Canthaxanthin, Citranaxanthin, Echinenon, Lutein, Lycopin und Zeaxanthin.

10. Verfahren nach Anspruch 8, **dadurch gekennzeichnet, dass** man
a) ein oder mehrere Carotinoide in einem mit Wasser mischbaren, organischen Lösungsmittel oder einer Mischung aus Wasser und einem mit Wasser mischbaren, organischen Lösungsmittel bei Temperaturen größer 30°C löst,
b) die erhaltene Lösung mit einer wäßrigen Casein- oder Caseinat-Lösung mischt,
c) das Casein oder Caseinat zusammen mit dem Carotinoid bei einem pH-Wert der Dispersion ausflockt, der im Bereich von einer pH-Wert Einheit oberhalb und unterhalb des isoelektrischen Punktes von Casein oder Caseinat liegt,
d) den ausgeflockten Feststoff vom Wasser und vom Lösungsmittel abtrennt und trocknet.

11. Feste Zubereitungen mindestens eines, für den Lebensmittel- und Tierfuttermittelbereich oder für pharmazeutische und kosmetische Anwendungen geeigneten, wasserlöslichen, schwer wasserlöslichen oder wasserunlöslichen Wirkstoffs, ausgewählt aus der Gruppe, bestehend aus fettlöslichen Vitaminen, wasserlöslichen Vitaminen, mehrfach ungesättigten Fettsäuren, Carotinoiden und organischen UV-Filtersubstanzen, erhältlich nach einem Verfahren, definiert gemäß einem der Ansprüche 1 bis 10.

12. Feste Zubereitungen nach Anspruch 11, enthaltend mindestens einen, für den Lebensmittel- und Tierfuttermittelbereich oder für pharmazeutische und kosmetische Anwendungen geeigneten, schwer wasserlöslichen oder wasserunlöslichen Wirkstoff, ausgewählt aus der Gruppe, bestehend aus fettlöslichen Vitaminen, wasserlöslichen Vitaminen, mehrfach ungesättigten Fettsäuren, Carotinoide und organische UV-Filtersubstanzen.

13. Feste Zubereitungen nach einem der Ansprüche 11 oder 12 mit einem Wirkstoffgehalt von 0,1 bis 80 Gew.-%.

14. Feste Zubereitungen nach einem der Ansprüche 12 oder 13, **dadurch gekennzeichnet, dass** es sich um Carotinoid-haltige Trockenpulver handelt.

15. Trockenpulver nach Anspruch 14, enthaltend Carotinoide, ausgewählt aus der Gruppe, bestehend aus Astaxanthin, β-Carotin, β-Apo-8'-Carotinal, β-Apo-8'-Carotinsäureethylester, Canthaxanthin, Citranaxanthin, Echinenon, Lutein, Lycopin und Zeaxanthin.

16. Verwendung der festen Zubereitungen, definiert gemäß Anspruch 11, als Zusatz zu Lebensmitteln, Tierfuttermitteln, Pharmazeutika und/oder kosmetischen Zubereitungen.

17. Verwendung nach Anspruch 16, **dadurch gekennzeichnet, dass** man die festen Zubereitungen in Form öliger Suspensionen einsetzt.

18. Verwendung der festen Zubereitungen, definiert gemäß Anspruch 11, zur Herstellung öliger Suspensionen.

19. Ölige Suspensionen, enthaltend als disperse Phase feste Zubereitungen mindestens eines, für den Lebensmittel- und Tierfuttermittelbereich oder für pharmazeutische und kosmetische Anwendungen geeigneten, wasserlöslichen, schwer wasserlöslichen oder wasserunlöslichen Wirkstoffs, ausgewählt aus der Gruppe, bestehend aus fettlöslichen Vitaminen, wasserlöslichen Vitaminen, mehrfach ungesättigten Fettsäuren, Carotinoiden und organischen UV-Filtersubstanzen, die erhältlich sind durch
a) Lösen oder Dispergieren mindestens eines der oben genannten Wirkstoffe in einer wäßrigen molekulardispersen oder kolloiddispersen Lösung eines proteinhaltigen Schutzkolloids,
b) Ausflockung des proteinhaltigen Schutzkolloids zusammen mit dem Wirkstoff aus der Dispersion durch Einstellung des pH-Wertes der Dispersion auf einen Wert, der im Bereich von einer pH-Wert Einheit oberhalb und unterhalb des isoelektrischen Punktes des als Schutzkolloid verwendeten Proteins liegt
und
c) Abtrennung des ausgeflockten Feststoffs vom Wasser und von gegebenenfalls zusätzlich verwendeten Lösungsmitteln und anschließendes Überführen in ein Trockenpulver,
wobei die Suspension einen Wassergehalt von 0,2 bis 4 Gew.-% aufweisen.

20. Ölige Suspensionen nach Anspruch 19 mit einem Wirkstoffgehalt von 0,1 bis 50 Gew.-%, bezogen auf die Gesamtmenge der öligen Suspension.

21. Ölige Suspensionen nach einem der Ansprüche 19 oder 20, enthaltend als Wirkstoff mindestens ein Carotinoid, ausgewählt aus der Gruppe, bestehend aus Astaxanthin, β-Carotin, β-Apo-8'-Carotinal, β-Apo-8'-Carotinsäureethylester, Canthaxanthin, Citranaxanthin, Echinenon, Lutein, Lycopin und Zeaxanthin.

22. Verfahren zur Herstellung Carotinoid-haltiger öliger Suspensionen, definiert gemäß Anspruch 19, **dadurch gekennzeichnet, dass** man
a) ein Trockenpulver, enthaltend mindestens ein von einem oder mehreren Schutzkolloiden umhülltes Carotinoid in mindestens einem Öl bis auf eine mittlere Partikelgröße von 0,1 bis 100 µm mahlt oder
b) ein Trockenpulver, enthaltend mindestens ein von einem oder mehreren Schutzkolloiden umhülltes Carotinoid ohne Verwendung einer kontinuierlichen Phase bis auf eine mittlere Partikelgröße von 0,1 bis 100 µm mahlt und die gemahlenen Partikel anschließend in mindestens einem Öl suspendiert oder
c) eine Carotinoid-haltige Suspension, enthaltend als feste Phase mindestens ein von einem oder mehreren Schutzkolloiden umhülltes Carotinoid und als Dispersionsmittel Wasser oder ein Gemisch aus Wasser und einem wassermischbaren Lösungsmittel, bis auf eine mittlere Partikelgröße von 0,1 bis 100 µm mahlt, die feste Phase anschließend vom Wasser bzw. vom Wasser/Lösungsmittel-Gemisch befreit und die so erhaltenen gemahlenen Partikel in mindestens einem Öl suspendiert.

23. Verfahren nach Anspruch 22, **dadurch gekennzeichnet, dass** es sich bei dem Öl um ein bei 20°C flüssiges, essbares Öl handelt.

24. Verfahren nach Anspruch 22, **dadurch gekennzeichnet, dass** es sich bei dem Öl um ein bei 20°C festes Hartfett handelt.

25. Verwendung der öligen Suspensionen, definiert gemäß Anspruch einem der Ansprüche 19 bis 21 zur Herstellung von Nahrungsergänzungsmitteln sowie als Zusatz zu Tierfuttermitteln, Lebensmitteln, pharmazeutischen und kosmetischen Zubereitungen.

26. Verwendung nach Anspruch 25, **dadurch gekennzeichnet, dass** es sich um Carotinoid-haltige ölige Suspensionen handelt.

27. Verwendung nach einem der Ansprüche 25 oder 26 als Futtermittelzusatz in der Tierernährung.

28. Verwendung nach Anspruch 27 zum Einmischen in Futtermittelpellets.

29. Verwendung nach Anspruch 28 zum Auftragen auf Futtermittelpellets.

30. Verwendung nach Anspruch 29, **dadurch gekennzeichnet, dass** man die Futtermittelpellets unter vermindertem Druck mit der öligen Suspension belädt.

31. Nahrungsergänzungsmittel, Tierfuttermittel, Lebensmittel sowie pharmazeutische und kosmetische Zubereitungen, enthaltend ölige Suspensionen, definiert gemäß einem der Ansprüche 19 bis 21.

## Claims

1. A process for producing solid preparations of at least one water-soluble, sparingly water-soluble or water-insoluble active compound suitable for the food and animal feed sectors or for pharmaceutical and cosmetic applications selected from the group consisting of fat-soluble vitamins, water-soluble vitamins, polyunsaturated fatty acids, carotenoids and organic UV filter substances, by
a) dissolving or dispersing at least one of the abovementioned active compounds in an aqueous molecular dispersion or colloidal dispersion of a proteinaceous protecting colloid,
b) flocculating the proteinaceous protecting colloid together with the active compound out of the dispersion
and
c) separating off the flocculated solid from the water and any solvents additionally used and subsequently converting them into a dry powder,
wherein, in process step b), the flocculation is initiated by setting the pH of the dispersion to a value which is in the range of one pH unit above and below the isoelectric point of the protein used as protecting colloid.

2. The process according to claim 1 for producing solid preparations of at least one sparingly water-soluble or water-insoluble active compound suitable for the food and animal feed sectors or for pharmaceutical and cosmetic applications, wherein, in process step a), at least one of the abovementioned active compounds is dispersed in an aqueous molecular dispersion or colloidal dispersion of a proteinaceous protecting colloid.

3. The process according to claim 2, wherein the dispersion step a) is the production of a suspension of at least one solid active compound in an aqueous molecular dispersion or colloidal dispersion of a proteinaceous protecting colloid.

4. The process according to claim 3, wherein the suspension produced in process step a) is ground before the flocculation.

5. The process according to claim 2, wherein the dispersion in stage a) comprises the following steps:
a₁) dissolving one or more sparingly water-soluble or water-insoluble active compounds selected from the group consisting of fat-soluble vitamins, polyunsaturated fatty acids, carotenoids and organic UV filter substances, in a water-miscible organic solvent or a mixture of water and a water-miscible organic solvent or
a₂) dissolving one or more sparingly water-soluble or water-insoluble active compounds selected from the group consisting of fat-soluble vitamins, polyunsaturated fatty acids, carotenoids and organic UV filter substances, in a water-immiscible organic solvent and
a₃) mixing the solution obtained by a₁) or a₂) with an aqueous molecular dispersion or colloidal dispersion of a proteinaceous protecting colloid, the hydrophobic phase of the active compound being produced as nanodisperse phase.

6. The process according to claim 5, wherein, when process step a₂) is being performed, the water-immiscible solvent is distilled off before flocculating the protecting colloid.

7. The process according to one of claims 1 to 6, wherein the protecting colloid is casein or a caseinate.

8. The process according to one of claims 1 to 7, which involves the production of carotenoid-containing dry powders.

9. The process according to claim 8 for producing dry powders comprising carotenoids selected from the group consisting of astaxanthin, β-carotene, β-apo-8'-carotenal, β-apo-8'-carotenic acid ethyl ester, canthaxanthin, citranaxanthin, echinenone, lutein, lycopene and zeaxanthin.

10. The process according to claim 8, wherein
a) one or more carotenoids are dissolved in a water-miscible organic solvent, or a mixture of water and a water-miscible organic solvent, at temperatures above 30°C,
b) the resultant solution is mixed with an aqueous casein solution or caseinate solution,
c) the casein or caseinate is flocculated out of the dispersion together with the carotenoid at a pH of the dispersion which is in the region of a pH unit above and below the isoelectric point of casein or caseinate,
d) the flocculated solid is separated off from the water and solvent and dried.

11. The solid preparation of at least one water-soluble, sparingly water-soluble or water-insoluble active compound suitable for the food and animal feed sectors or for pharmaceutical and cosmetic applications selected from the group consisting of fat-soluble vitamins, water-soluble vitamins, polyunsaturated fatty acids, carotenoids and organic UV filter substances, and obtainable by a process as defined in one of claims 1 to 10.

12. The solid preparation according to claim 11 comprising at least one sparingly water-soluble or water-insoluble active compound suitable for the food and animal feed sectors or for pharmaceutical and cosmetic applications selected from the group consisting of fat-soluble vitamins, water-soluble vitamins, polyunsaturated fatty acids, carotenoids and organic UV filter substances.

13. The solid preparation according to either of claims 11 or 12 having an active compound content of from 0.1 to 80% by weight.

14. The solid preparation according to either of claims 12 or 13 which is a carotenoid-containing dry powder.

15. The dry powder according to claim 14 comprising carotenoids selected from the group consisting of astaxanthin, β-carotene, β-apo-8'-carotenal, β-apo-8'-carotenic acid ethyl ester, canthaxanthin, citranaxanthin, echinenone, lutein, lycopene and zeaxanthin.

16. The use of the solid preparation as defined in claim 11 as additive to foods, animal feeds, pharmaceuticals and/or cosmetic preparations.

17. The use according to claim 16, wherein the solid preparation is used in the form of an oily suspension.

18. The use of the solid preparation as defined in claim 11 for producing oily suspensions.

19. An oily suspension comprising, as disperse phase, solid preparations of at least one water-soluble, sparingly water-soluble or water-insoluble active compound suitable for the food and animal feed sectors or for pharmaceutical and cosmetic applications selected from the group consisting of fat-soluble vitamins, water-soluble vitamins, polyunsaturated fatty acids, carotenoids and organic UV filter substances, which is obtainable by
a) dissolving or dispersing at least one of the abovementioned active compounds in an aqueous molecular dispersion or colloidal dispersion of a proteinaceous protecting colloid,
b) flocculating the proteinaceous protecting colloid together with the active compound out of the dispersion, by setting the pH of the dispersion to a value which is in the range of one pH unit above and below the isoelectric point of the protein used as protecting colloid,
and
c) separating off the flocculated solid from the water and any solvents additionally used and subsequently converting them into a dry powder,
wherein the suspension has a water content of 0.2 to 4% by weight.

20. The oily suspension according to claim 19 having an active compound content of from 0.1 to 50% by weight, based on the total amount of oily suspension.

21. The oily suspension according to either of claims 19 or 20 comprising, as active compound, at least one carotenoid selected from the group consisting of astaxanthin, β-carotene, β-apo-8'-carotenal, β-apo-8'-carotenic acid ethyl ester, canthaxanthin, citranaxanthin, echinenone, lutein, lycopene and zeaxanthin.

22. A process for producing a carotenoid-containing oily suspension as defined in claim 19, which comprises
a) grinding a dry powder comprising at least one carotenoid enclosed by one or more protecting colloids in at least one oil to a mean particle size of from 0.1 to 100 µm or
b) grinding a dry powder comprising at least one carotenoid enclosed by one or more protecting colloids without using a continuous phase to a mean particle size of from 0.1 to 100 µm and then suspending the ground particles in at least one oil or
c) grinding a carotenoid-containing suspension comprising, as solid phase, at least one carotenoid enclosed by one or more protecting colloids and, as dispersion medium, water or a mixture of water and a water-miscible solvent to a mean particle size of from 0.1 to 100 µm, then freeing the solid phase from the water or water/solvent mixture and suspending the resultant ground particles in at least one oil.

23. The process according to claim 22, wherein the oil is an edible oil liquid at 20°C.

24. The process according to claim 22, wherein the oil is a hard fat solid at 20°C.

25. The use of the oily suspension as defined in one of claims 19 to 21 for producing food supplements and as additive to animal feeds, foods, pharmaceutical and cosmetic preparations.

26. The use according to claim 25 wherein the oily suspension comprises carotenoid.

27. The use according to either of claims 25 or 26 as feed additive in animal nutrition.

28. The use according to claim 27 for incorporation into feed pellets.

29. The use according to claim 28 for application to food pellets.

30. The use according to claim 29, wherein the feed pellets are charged with the oily suspension at reduced pressure.

31. A food supplement, animal feed, food or pharmaceutical or cosmetic preparation comprising an oily suspension as defined in one of claims 19 to 21.

## Revendications

1. Procédé de préparation de compositions solides d'au moins une substance active soluble dans l'eau, difficilement soluble dans l'eau ou insoluble dans l'eau qui est appropriée dans le domaine des aliments et des fourrages pour animaux ou pour des applications pharmaceutiques et cosmétiques et qui est choisie parmi le groupe constitué des vitamines liposolubles, des vitamines solubles dans l'eau, des acides gras plusieurs fois insaturés, des caroténoïdes et des substances organiques filtrant les U.V., par
a) dissolution ou dispersion d'au moins une des substances actives précitées dans une solution aqueuse à dispersion moléculaire ou colloïdale d'un colloïde de protection protéinique,
b) floculation dans la dispersion du colloïde de protection protéinique conjointement à la substance active,
et
c) séparation entre la substance solide floculée et l'eau et des solvants éventuellement utilisés en supplément et conversion ultérieure en une poudre sèche,
**caractérisé en ce que**, dans l'étape b) du procédé, on déclenche la floculation par ajustement de la valeur du pH de la dispersion à une valeur qui se trouve dans la gamme d'une unité de valeur de pH au-dessus et en dessous du point isoélectrique de la protéine utilisée comme colloïde de protection.

2. Procédé suivant la revendication 1, pour la préparation de compositions solides d'au moins une substance active difficilement soluble ou insoluble dans l'eau qui est appropriée dans le domaine des aliments et des fourrages pour animaux ou pour des applications pharmaceutiques et cosmétiques, **caractérisé en ce que**, dans l'étape a) du procédé, on disperse au moins une des substances actives précitées dans une solution aqueuse à dispersion moléculaire ou colloïdale d'un colloïde de protection protéinique.

3. Procédé suivant la revendication 2, **caractérisé en ce que**, en ce qui concerne l'étape de dispersion a), il s'agit de la préparation d'une suspension d'au moins une substance active solide dans une solution aqueuse à dispersion moléculaire ou colloïdale d'un colloïde de protection protéinique.

4. Procédé suivant la revendication 3, **caractérisé en ce qu'**on broie la suspension préparée dans l'étape a) du procédé avant la floculation.

5. Procédé suivant la revendication 2, **caractérisé en ce que** la dispersion de l'étape a) contient les phases suivantes :
a₁) une dissolution d'une ou de plusieurs substances actives difficilement solubles ou insolubles dans l'eau, choisies parmi le groupe constitué de vitamines liposolubles, d'acides gras plusieurs fois insaturés, de caroténoïdes et de substances organiques filtrant les U.V., dans un solvant organique miscible à l'eau ou un mélange d'eau et d'un solvant organique miscible à l'eau, ou
a₂) une dissolution d'une ou de plusieurs substances actives difficilement solubles ou insolubles dans l'eau, choisies parmi le groupe constitué des vitamines liposolubles, des acides gras plusieurs fois insaturés, des caroténoïdes et des substances organiques filtrant les U.V. dans un solvant organique non miscible à l'eau, et
a₃) un mélange de la solution obtenue selon a₁) ou a₂) avec une solution aqueuse à dispersion moléculaire ou colloïdale d'un colloïde de protection protéinique, la phase hydrophobe de la substance active étant obtenue sous la forme d'une phase nanodispersée.

6. Procédé suivant la revendication 5, **caractérisé en ce que**, lorsqu'on effectue l'étape a₂) du procédé, on sépare par distillation le solvant non miscible à l'eau avant la floculation du colloïde de protection.

7. Procédé suivant l'une des revendications 1 à 6, **caractérisé en ce que**, comme colloïde de protection, on utilise de la caséine ou un caséinate.

8. Procédé suivant l'une des revendications 1 à 7, **caractérisé en ce qu'**il s'agit de la préparation d'une poudre sèche contenant du caroténoïde.

9. Procédé suivant la revendication 8 pour la préparation de poudres sèches contenant des caroténoïdes, choisis parmi le groupe constitué d'astaxanthine, de β-caroténe, de β-apo-8'-caroténal, de β-apo-8'-caroténate d'éthyle, de canthaxanthine, de citranaxanthine, d'échinénone, de lutéine, de lycopine et de zéaxanthine.

10. Procédé suivant la revendication 8, **caractérisé en ce que**
a) à des températures supérieures à 30°C on dissout un ou plusieurs caroténoïdes dans un solvant organique miscible à l'eau ou un mélange d'eau et d'un solvant organique miscible à l'eau,
b) on mélange la solution obtenue avec une solution aqueuse de caséine ou de caséinate,
c) on flocule la caséine ou le caséinate conjointement avec le caroténoïde à une valeur de pH de la dispersion qui se trouve dans la gamme d'une unité de valeur de pH au-dessus et en dessous du point isoélectrique de la caséine ou du caséinate,
d) on sépare la substance solide floculée de l'eau et du solvant et on la sèche.

11. Compositions solides d'au moins une substance active soluble dans l'eau, difficilement soluble dans l'eau ou insoluble dans l'eau, qui est appropriée dans le domaine des aliments et des fourrages pour animaux ou pour des applications pharmaceutiques et cosmétiques, et qui est choisie parmi le groupe constitué de vitamines liposolubles, de vitamines solubles dans l'eau, d'acides gras plusieurs fois insaturés, de caroténoïdes et de substances organiques filtrant les U.V., que l'on peut obtenir selon un procédé défini conformément à une des revendications 1 à 10.

12. Compositions solides suivant la revendication 11, contenant au moins une substance active difficilement soluble ou insoluble dans l'eau qui est appropriée dans le domaine des aliments et des fourrages pour animaux ou pour des applications pharmaceutiques et cosmétiques et qui est choisie parmi le groupe constitué de vitamines liposolubles, de vitamines solubles dans l'eau, d'acides gras plusieurs fois insaturés, de caroténoïdes et de substances organiques filtrant les U.V.

13. Compositions solides suivant l'une des revendications 11 ou 12, ayant une teneur en substance active de 0,1 à 80 % en poids.

14. Compositions solides suivant l'une des revendications 12 ou 13, **caractérisées en ce qu'**il s'agit de poudres sèches contenant du caroténoïde.

15. Poudre sèche suivant la revendication 14, contenant des caroténoïdes choisis parmi le groupe constitué d'astaxanthine, de β-carotène, de β-apo-8'-caroténal, de β-apo-8'-caroténate d'éthyle, de canthaxanthine, de citranaxanthine, d'échinénone, de lutéine, de lycopine et de zéaxanthine.

16. Utilisation des compositions solides définies suivant la revendication 11, comme additif pour des aliments, des fourrages pour animaux, des produits pharmaceutiques et/ou des compositions cosmétiques.

17. Utilisation suivant la revendication 16, **caractérisée en ce qu'**on met en oeuvre les compositions solides sous la forme de suspensions huileuses.

18. Utilisation des compositions solides définies suivant la revendication 11, pour la préparation de suspensions huileuses.

19. Suspensions huileuses contenant, comme phase dispersée, des compositions solides d'au moins une substance active soluble dans l'eau, difficilement soluble dans l'eau ou insoluble dans l'eau, qui est appropriée pour le domaine des aliments et des fourrages pour animaux ou pour des applications pharmaceutiques et cosmétiques et qui est choisie parmi le groupe constitué de vitamines liposolubles, de vitamines solubles dans l'eau, d'acides gras plusieurs fois insaturés, de caroténoïdes et de substances organiques filtrant les U.V., qu'on peut obtenir par
a) dissolution ou dispersion d'au moins une des substances actives précitées dans une solution aqueuse à dispersion moléculaire ou colloïdale d'un colloïde de protection protéinique,
b) floculation dans la dispersion du colloïde de protection protéinique conjointement à la substance active, par ajustement de la valeur du pH de la dispersion à une valeur qui se trouve dans la gamme d'une unité de valeur de pH au-dessus et en dessous du point isoélectrique de la protéine utilisée comme colloïde de protection,
et
c) séparation entre la substance solide floculée et l'eau et les solvants éventuellement utilisés en supplément, et conversion ultérieure en une poudre sèche,
la suspension présentant une teneur en eau de 0,2 à 4 % en poids.

20. Suspensions huileuses suivant la revendication 19, présentant une teneur en substance active de 0,1 à 50 % en poids, par rapport à la quantité totale de la suspension huileuse.

21. Suspensions huileuses suivant l'une des revendications 19 ou 20, contenant comme substance active au moins un caroténoïde choisi parmi le groupe constitué d'astaxanthine, de β-carotène, de β-apo-8'-caroténal, de β-apo-8'-caroténate d'éthyle, de canthaxanthine, de citranaxanthine, d'échinénone, de lutéine, de lycopine et de zéaxanthine.

22. Procédé de préparation de suspensions huileuses contenant du caroténoïde, définies selon la revendication 19, **caractérisé en ce que**
a) on broie une poudre sèche contenant au moins un caroténoïde enveloppé d'un ou plusieurs colloïdes de protection dans au moins une huile jusqu'à obtention d'une taille moyenne de particule de 0,1 à 100 µm, ou
b) on broie une poudre sèche contenant au moins un caroténoïde enveloppé d'un ou de plusieurs colloïdes de protection sans utilisation d'une phase continue jusqu'à une taille moyenne de particule de 0,1 à 100 µm et on met ensuite les particules broyées en suspension dans au moins une huile, ou
c) on broie une suspension contenant du caroténoïde comportant, comme phase solide, au moins un caroténoïde enveloppé d'un ou de plusieurs colloïdes de protection et, comme agent dispersant, de l'eau ou un mélange d'eau et d'un solvant miscible à l'eau, jusqu'à obtention d'une taille moyenne de particule de 0,1 à 100 µm, on libère ensuite la phase solide de l'eau ou du mélange eau/solvant et on met les particules broyées ainsi obtenues en suspension dans au moins une huile.

23. Procédé suivant la revendication 22, **caractérisé en ce que**, pour ce qui concerne l'huile, il s'agit d'une huile comestible, fluide à 20°C.

24. Procédé suivant la revendication 22, **caractérisé en ce que**, pour ce qui concerne l'huile, il s'agit d'une graisse dure, solide à 20°C.

25. Utilisation des suspensions huileuses définies suivant l'une des revendications 19 à 21, pour la préparation de compléments alimentaires ainsi que comme additifs pour des fourrages pour animaux, des aliments, des compositions pharmaceutiques et cosmétiques.

26. Utilisation suivant la revendication 25, **caractérisée en ce qu'**il s'agit de suspensions huileuses contenant du caroténoïde.

27. Utilisation suivant l'une des revendications 25 ou 26, comme additif de fourrage dans l'alimentation des animaux.

28. Utilisation suivant la revendication 27 pour l'incorporation dans des pellets alimentaires pour animaux.

29. Utilisation suivant la revendication 28 pour l'application sur des pellets alimentaires pour animaux.

30. Utilisation suivant la revendication 29, **caractérisée en ce que**, sous pression réduite, on charge les pellets alimentaires pour animaux de la suspension huileuse.

31. Compléments alimentaires, fourrages pour animaux, aliments ainsi que compositions pharmaceutiques et cosmétiques contenant des suspensions huileuses définies suivant l'une des revendications 19 à 21.
